# FASCICULE DE BREVET EUROPEEN

(11) **EP 0 986 568 B1**
(45) Date de publication et mention de la délivrance du brevet: **20.03.2002**
(21) Numéro de dépôt: 98928418.7
(22) Date de dépôt: 03.06.1998
(51) Int. Cl.: C07H 15/18, C07H 15/04, A61K 9/127, A61K 48/00, C12N 15/88

(54) **NOUVELLE CLASSE D'AGENTS TRANSFECTANTS CATIONIQUES DES ACIDES NUCLEIQUES**
KATIONISCHE MITTEL ZUR TRANSFEKTION VON NUKLEINSÄUREN
NOVEL CLASS OF NUCLEIC ACID CATIONIC TRANSFECTING AGENTS

(30) Priorité: 06.06.1997 FR 9707014
(43) Date de publication de la demande: 22.03.2000
(73) Titulaire: Aventis Pharma S.A., 92160 Antony (FR)
(72) Inventeur: BESSODES, Michel, F-94800 Villejuif (FR); SCHERMAN, Daniel, F-75012 Paris (FR); HERSCOVICI, Jean, F-75013 Paris (FR)
(86) Numéro de dépôt international: FR9801112
(87) Numéro de publication internationale: WO9855490

(56) Documents cités:
- WO-A-96/24334
- DE-A- 4 329 093
- CHEMICAL ABSTRACTS, vol. 120, no. 13, 28 mars 1994 Columbus, Ohio, US; abstract no. 164909, XP002065370 & JP 05 202 085 A (DEI DEI ESU KENKYUSHO KK)

## Description

La présente invention se rapporte à une nouvelle classe d'agents transfectants cationiques, aux compositions pharmaceutiques les contenant, leurs applications pour la transfection *in vivo, ex vivo* et/ou *in vitro* d'acides nucléiques et leurs procédés de préparation.

Avec le développement des biotechnologies, il est désormais possible de transférer des acides nucléiques dans les cellules. L'efficacité de ces transferts apparaît nécessaire pour la correction de défauts d'expression et/ou d'expression anormale d'acides nucléiques impliqués dans de nombreuses maladies génétiques. Cependant, l'intérêt des tranferts d'acides nucléiques ne se limite pas à la thérapie génique. En outre, le transfert d'acides nucléiques peut être utile pour l'étude de la régulation de l'expression de gènes, le clonage de gènes, ou pour toute autre manipulation *in vitro* impliquant les acides nucléiques, aussi bien que pour la production de protéines recombinantes. Il peut également s'agir du transfert d'acides nucléiques *in vivo,* par exemple pour l'obtention d'animaux transgéniques, la réalisation de vaccins, des études de marquage de molécules. Par ailleurs, le transfert d'acides nucléiques peut être réalisé dans des cellules *ex vivo*, dans des approches de greffes de moelle osseuse, d'immunothérapie ou d'autres méthodes impliquant le transfert de gènes dans des cellules prélevées d'un organisme en vue d'être réadministrées ultérieurement.

Aujourd'hui, plusieurs méthodes sont proposées pour la délivrance intracellulaire de ce type d'information génétique. L'une d'entre elles, en particulier, repose sur l'emploi de vecteurs chimiques ou biochimiques. Ces vecteurs synthétiques ont deux fonctions principales : complexer l'ADN à transfecter et promouvoir sa fixation cellulaire ainsi que son passage à travers la membrane plasmique et, le cas échéant, à travers les deux membranes nucléaires. Parmi les vecteurs synthétiques développés, les polymères cationiques de type polylysine et DEAE dextran ou encore les lipofectants sont les plus avantageux.

Un progrès important a été accompli dans ce mode de transfection avec le développement d'une technologie basée sur l'emploi d'agents de transfection cationiques de type lipofectants, et plus précisément de lipides cationiques. Il a ainsi été mis en évidence qu'un lipide cationique chargé positivement, le chlorure de N-[1-(2,3-dioléyloxy)propyl]-N,N,N-triméthylammonium (DOTMA), interférait, sous la forme de liposomes ou de petites vésicules, spontanément avec de l'ADN, qui est chargé négativement, pour former des complexes lipides-ADN, capables de fusionner avec les membranes cellulaires, et permettait ainsi la délivrance intracellulaire de l'ADN.

Depuis le DOTMA, d'autres lipides cationiques ont été développés sur ce même modèle de structure, c'est-à-dire un groupe lipophile couplé à un groupement amino via un bras encore appelé "spacer". Parmi ceux-ci, on peut plus particulièrement citer ceux comprenant à titre de groupement lipophile des acides gras ou un dérivé du cholestérol, et comportant en outre, le cas échéant, à titre de groupement amino, un groupement d'ammonium quaternaire. Le DOTAP, le DOBT ou le ChOTB peuvent notamment être cités à titre représentatifs de cette catégorie de lipides cationiques. D'autres composés, comme le DOSC et le ChOSC, se caractérisent par la présence d'un groupement choline à la place du groupement d'ammonium quaternaire.

Une autre catégorie de lipofectants, les lipopolyamines, a également été décrite. De manière générale, il s'agit d'une molécule amphiphile comprenant au moins une région hydrophile constituée par une polyamine couplée via un "spacer" à une région lipophile. La région polyaminée, chargée positivement, est capable de s'associer de manière réversible avec l'acide nucléique, chargé négativement. Cette interaction compacte fortement l'acide nucléique. Quant à la région lipophile, elle rend cette interaction ionique insensible au milieu externe, en recouvrant le complexe formé d'une pellicule lipidique. Dans ce type de composés, le groupement cationique peut être représenté par le radical L-5-carboxyspermine qui contient quatre groupements d'ammonium, deux primaires et deux secondaires. Le DOGS et le DPPES en font notamment partie. Ces lipopolyamines sont tout particulièrement efficaces pour la transfection de cellules endocrines primaires. A titre représentatif de cette dernière famille de composés on peut plus particulièrement mentionner les lipopolyamines décrites par exemple dans les demandes de brevet WO 96/17823 et WO 97/18185.

Toutefois, l'efficacité de ces vecteurs synthétiques reste à améliorer notamment en terme de densité de charge et de rigidité de la molécule transfectante.

En effet, il est généralement admis que l'activité de ces produits dépend de la densité de charge qu'ils font intervenir. Cependant, l'augmentation des charges sur des chaînes aliphatiques est à l'origine de l'apparition de la toxicité. Une stabilisation de la densité de charge des chaînes aliphatiques préviendrait donc l'apparition de facteurs toxiques. De plus, l'efficacité de la transfection serait accrue par une augmentation de la densité de charge dans une région autre que les chaînes aliphatiques.

Par ailleurs, la flexibilité de la molécule utilisée pour le transfert d'un acide nucléique peut être un obstacle à une interaction suffisamment étroite entre le dit acide nucléique et l'agent transfectant, empêchant d'obtenir un compactage optimal de la molécule d'acide nucléique, le compactage étant une condition préalable nécessaire à toute transfection. Une rigidité accrue de la molécule d'agent tranfectant asssurerait une interaction plus efficace avec l'acide nucléique, améliorant ainsi la transfection.

Ainsi, il serait particulièrement intéressant de disposer d'agents tranfectants présentant une capacité de transfection accrue tout en possédant une toxicité réduite ou nulle. Ce sont ces objectifs que la présente invention se propose d'atteindre.

La présente invention a en effet précisément pour objectif de proposer une nouvelle classe d'agents transfectants présentant une région hydrophile cationique originale qui confère aux dits agents de transfert les propriétés particulières mentionnées ci-dessus.

Plus précisément, la présente invention se rapporte à un agent transfectant comprenant au moins une région hydrophile cationique couplée à une région lipophile, la dite région hydrophile cationique étant constituée d'au moins un hétérocycle à 5 ou 6 atomes substitué par des groupements amino.

Le brevet DE 4329093 décrit des (ω-aminoalkyl)glycosides qui ont pour fonction de stimuler les défenses immunitaires. Aucune de ces structures n'est cependant décrite comme agent de transfection d'acides nucléiques.

La région hydrophile chargée positivement est capable de s'associer de manière réversible à un acide nucléique, chargé négativemenent, qui est ainsi fortement compacté. En outre, la structure originale de la partie cationique confère à la molécule une rigidité accrue.

De préférence, la présente invention concerne des agents transfectants comprenant au moins une région hydrophile cationique couplée à une région lipophile caractérisé en ce que ladite région hydrophile cationique a pour formule générale : 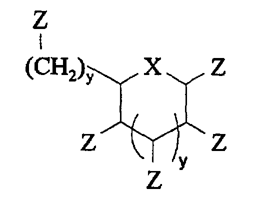 dans laquelle :
- y est un nombre entier égal à 0 ou 1, les différents y étant indépendants les uns des autres,
- X représente un atome d'oxygène, d'azote, de soufre ou de sélénium,
- les groupements Z représentent, indépendamment les uns des autres,
   - un atome d'hydrogène,
   - un groupement OR dans lequel R représente un atome d'hydrogène, un groupement méthyle, ou un groupement (CH2)ₙ-NR₁R₂ dans lequel n est un nombre entier choisi de 1 à 6 inclus et R₁ et R₂ représentent, indépendamment l'un de l'autre, un atome d'hydrogène ou un groupement (CH₂)q-NH₂, q pouvant varier de 1 à 6 inclus, les différents q étant indépendants les uns des autres,
   - un groupement (CH₂)ₘ-NR₁R₂, dans lequel m est un nombre entier choisi de 0 à 6 inclus et R₁ et R₂ sont définis comme précédemment, ou bien
   - un groupement "spacer" permettant la liaison de la région hydrophile cationique à la région lipophile,
   étant entendu que l'un au moins des substituants Z est un groupement « spacer » et que au moins deux des substituants Z portent un groupement amino.

Les agents de transfert selon l'invention contiennent au moins une région hydrophile cationique de formule générale (I). Selon une variante de l'invention, les agents de transfert comprennent 2 ou plusieurs régions hydrophiles cationiques qui sont liées les unes aux autres au niveau de l'un des groupements Z.

Selon un mode de réalisation préféré de l'invention, lorsque l'un des substituants Z représente OR et R représente un groupement (CH₂)ₙ-NR₁R₂, alors n est préférentiellement choisi parmi 2, 3 ou 4.

Selon une variante avantageuse de l'invention, les agents transfectants comportent une région hydrophile cationique de formule générale (I) dans laquelle X représente un atome d'oxygène. La partie hydrophile cationique est alors constituée d'un glycoside de forme pyranose ou furanose. La forme pyranose est particulièrement intéressante et a été décrite de manière non limitative dans les exemples.

Selon une seconde variante avantageuse de la présente invention, les agents transfectants comprennent une région hydrophile cationique de formule générale (I) composée d'un glycoside aminé à 6 chaînons (y présent sur le cycle est égal à 1), X est un atome d'oxygène et au moins un des substituants Z comporte un groupement amino. Encore plus préférentiellement, au moins deux des substituants Z comportent un groupement amino.

Selon une autre variante de la présente invention, la région hydrophile cationique est composée d'un glycoside aminé de formule (I), dans laquelle les deux y sont égaux à 1, X est un atome d'oxygène, deux des groupements Z représentent des atomes d'hydrogène, deux autres groupements Z sont des groupements azotés et préférentiellement des groupements amino, et le dernier groupement Z représente un groupement OR, R étant défini comme précédement. De manière préférée, le groupement OR est situé en position -2 sur l'hétérocycle constituant la région hydrophile cationique des agents transfectants selon l'invention.

Selon un autre mode de réalisation avantageux de l'invention, la région hydrophile cationique est composée d'un glycoside aminé de formule générale (I), dans laquelle le y présent sur le cycle est égal à 1, X est un atome d'oxygène, au moins deux groupements Z correspondent à des groupements O-(CH₂)_{q}-NH₂ q étant défini comme précédemment, et au moins un des groupements Z représente un groupement OR, R étant défini comme précédement.

A titre représentatif de régions hydrophiles cationiques de formule générale (I), intermédiaires utiles dans la synthèse des agents de transfert selon l'invention, on peut citer plus particulièrement les composés suivants :

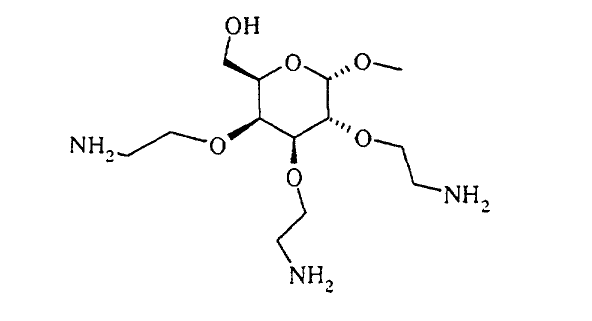

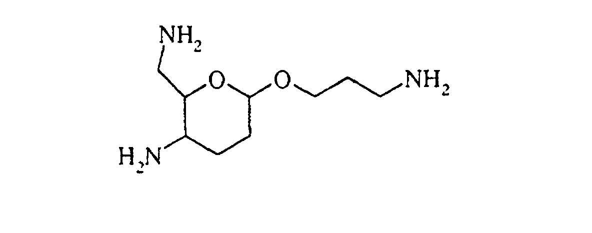

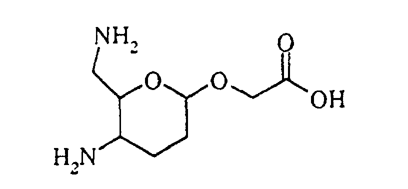

De manière générale, les agents transfectants au sens de la présente invention comportent au moins une région hydrophile cationique telle que définie précédemment associée à une région lipophile.

Les molécules constituants la région lipophile au sens de l'invention sont choisies parmi les molécules lipophiles connues de l'homme de métier. Avantageusement, la région lipophile est constituée par une ou plusieurs chaînes aliphatiques linéaires ou ramifiées, saturées ou non, éventuellement halogénées. La région lipophile peut également être avantageusement choisie parmi les dérivés de stéroïde.

Selon une variante préférée de l'invention, la partie lipophile est constituée d'une ou pusieurs chaînes aliphatiques contenant 10 à 22 atomes de carbone, et encore plus préférentiellement 12 à 22 atomes de carbone. A titre d'exemple, on peut citer les chaînes aliphatiques contenant 14, 16, 17, 18 ou 19 atomes de carbone, et notammant (CH₂)₁₃CH₃, (CH₂)₁₅CH₃, (CH₂)₁₆CH₃, (CH₂)₁₇CH₃, et (CH₂)₁₈CH₃.

La partie lipophile des agents de transfert selon l'invention peut aussi être avantageusement choisie parmi les dérivés de stéroïde comme par exemple le cholestérol, le cholestanol, le 3-α-5-cyclo-5-α-cholestan-6-β-ol, l'acide cholique, le cholestérylformiate, le cholestanylformiate, le 3α,5-cyclo-5α-cholestan-6β-yl formiate, la cholestérylamine, la 6-(1,5-diméthylhexyl) -3a,5a-diméthylhexadécahydrocyclopenta[a]cyclopropa[2,3]cyclopenta[1,2-f]naphta-lèn-10-ylamine, ou la cholestanylamine.

Selon un mode de réalisation préféré, la région hydrophile cationique est couplée à la région lipophile par une molécule intermédaire dite "spacer". On entend au sens de l'invention par groupement "spacer" tout substituant acide ou aminé comportant des fonctions hydrolysables qui permettent d'obtenir des liaisons amide, carbamate, ester, éther ou via un cycle aromatique entre la partie hydrophile cationique et la région lipophile, connu de l'homme du métier. Le couplage entre la partie hydrophile et la partie lipophile est préférentiellement opéré au niveau de l'un des groupements Z présent sur l'intermédiaire hydrophile cationique de formule (I) et utile pour la synthèse des agents de transfert selon l'invention. Avantageusement, le couplage entre les deux régions hydophile et lipophile, est effectué en position -2 ou en position -5 ou -6 (selon si y présent sur le cycle est égal à 0 ou 1) sur le cycle de la partie hydrophile cationique de formule générale (I), via un groupement "spacer".

De manière préférée, la région "spacer" comporte une chaîne aliphatique ou aromatique. En outre, le "spacer" comporte avantageusement un ou plusieurs groupements choisis parmi les groupements amide, carbamate, ester, éther ou les cycles aromatiques. Des "spacer" préférés sont notamment ceux de formule -O-CO-(CH₂)ₓ-COOH, -O-(CH₂)ₓ-COOH, -O-CO-(CH₂)ₓ-NH₂, -O-(CH₂)ₓ-NH₂, -NH-(CH₂)ₓ-NH₂, avec x représentant un entier choisi de 1 à 6 inclus.

A titre représentatif d'agents transfectants selon l'invention, on peut citer plus particulièrement les composés (16), (17), (8a) et (8b) de formules :

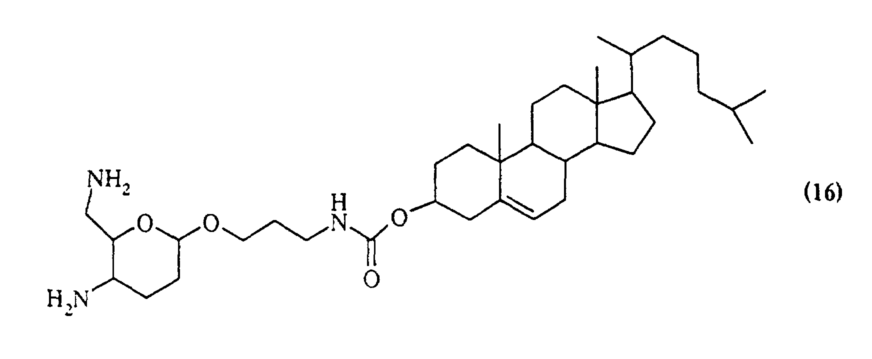

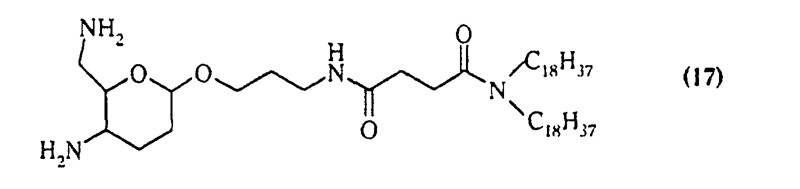

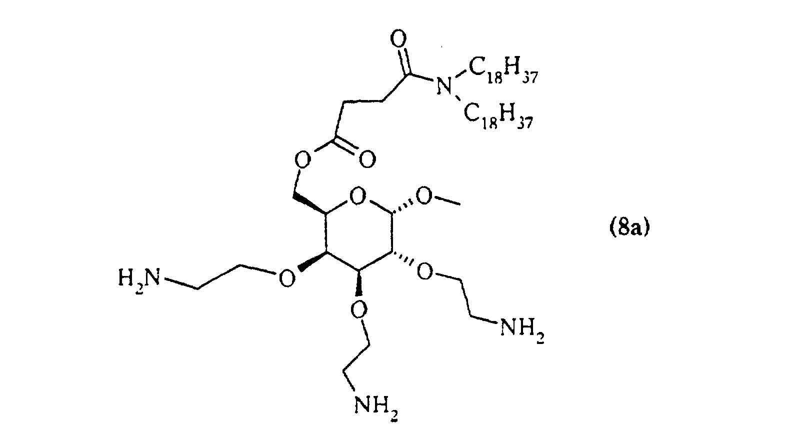

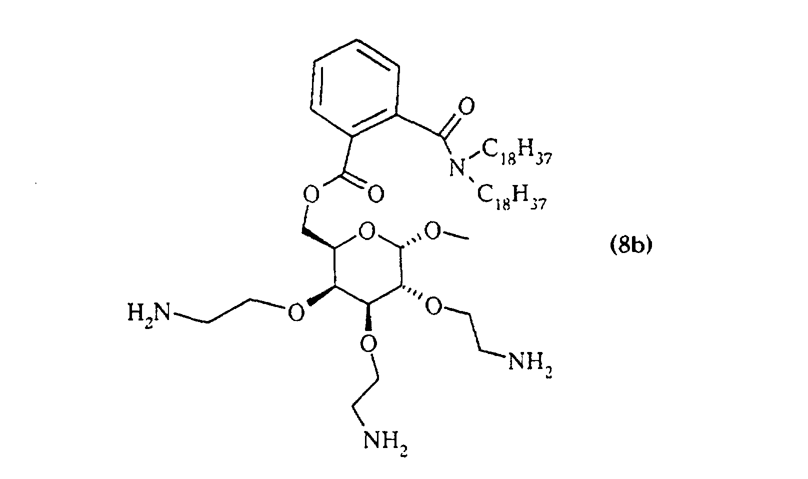

La présente invention se rapporte également aux procédés de préparation d'une nouvelle classe d'agents de transfection d'acides nucléiques tels que définis ci-dessus. Plus précisément, elle se rapporte aux procédés de préparation des dits agents tranfectants à partir d'un hétérocycle mono- ou polysubstitué auquel est couplé une région lipidique par l'intermédiaire d'un "spacer".

La région hydrophile cationique de formule générale (I) peut être préparée de différentes façons, selon la position relative des fonctions amino sur le cycle et leur nombre.

Lorsque l'on souhaite obtenir une région hydrophile cationique de formule générale (I) pour laquelle l'un au moins des substituants Z est un "spacer" permettant la liaison à la partie lipophile et l'un au moins des groupements Z est un groupement OR avec R représentant un groupement (CH₂)ₙ-NR₁R₂, on opère à partir du dérivé correspondant pour lequel tous les substituants O-(CH₂)ₙ-NR₁R₂ sont des fonctions hydroxy.

Dans une première étape, le dit dérivé portant les fonctions hydroxy subit une O-alkylation selon les méthodes classiques connues de l'homme du métier. Notamment, on opère à l'aide d'un agent alkylant en milieu basique dans les solvants classiques d'alkylation, en présence d'un éther couronne et à température comprise entre 10 et 60°C.

On utilise notamment à titre d'agent alkylant les alkylamines, les dérivés halogénés des esters, comme par exemple l'halogénoacétate d'alkyle, ou les dérivés halogénés des alcools. De préférence, on utilise du bromoacétate d'alkyle, les fonctions alkyle sont choisies en fonction de la valeur que l'on souhaite donner à n. Par exemple, pour avoir n égal à 2, on utilise de préférence du bromoacétate d'éthyle.

Les solvants utilisés sont les solvants classiques des O-alkylations, par exemple la diméthylformamide, la diméthylacétamide, le diméthyisuifoxyde, l'acétonitrile, le tetrahydrofurane (THF), etc.... Avantageusement, on utilise le THF.

La réaction est effectuée en présence d'une base, par exemple l'hydroxyde de potassium ou l'hydrure de sodium.

Dans une seconde étape, les fonctions carboxy du dérivé obtenu sont réduites en alcool selon les méthodes classiques connues de l'homme du métier. On opère notamment par action d'un agent réducteur dans des solvants classiques compatibles.

A titre d'agent réducteur, on cite par exemple le boranediméthylsulfure (BMS), l'hydrure de lithium aluminium ou le borohydrure de sodium.

Des solvants compatibles sont par exemple les éthers ou les alcools. De préférence, on utilise le tétrahydrofurane (THF).

Dans un troisième temps, les fonctions hydroxy obtenues sont azidées par des méthodes classiques connues de l'homme du métier.

On opère notamment par action de l'acide hydrazoïque en présence de triphénylphosphine et de diéthylazodicarboxylate dans un solvant compatible avec la réaction.

Les solvants utilisables sont les solvants classiques d'azidation comme par exemple le tétrahydrofurane, le benzène, le toluène, le chloroforme, le dichlorométhane, etc..., et de préférence le tétrahydrofurane (THF).

Enfin, les fonctions azides sont transformées en fonctions amino par les méthodes classiques connues de l'homme du métier.

On opère notamment par réduction en milieu acide, par exemple par hydrogénation en milieu acide en présence de palladium sur charbon, ou en mettant en oeuvre la réaction de Staudinger, ou encore par action d'un agent réducteur, par exemple l'hydrure de lithium aluminium, le borohydrure de sodium, le chlorure stanneux etc...

On obtient ainsi un composé hydrophyle cationique de formule générale (I) pour laquelle l'un au moins des substituants Z est un "spacer" permettant la liaison à la partie lipophile et l'un au moins des groupements Z est un groupement OR avec R représentant un groupement -(CH₂)ₙ-NR₁R₂.

Le couplage à la partie lipophile est ensuite effectué par les méthodes classiques connues de l'homme du métier, et notamment par couplage peptidique (Bodanski M., *Principles and Practices of Peptides Synthesis,* Ed. Springe-Verlag). Le couplage intervient au niveau du groupement Z représentant un "spacer".

Lorsque l'un des Z est un groupement "spacer", celui-ci est, le cas échéant, préalablement protégé. Il en va de même des fonctions amino portées par la partie hydrophile cationique qui sont de préférence protégées préalablement au couplage peptidique. La protection et l'élimination des radicaux protecteurs s'effectuent selon les méthodes habituelles.

La protection peut être réalisée par tout groupement compatible et dont la mise en oeuvre et l'élimination n'altère pas le reste de la molécule. Notamment, on opère selon les méthodes décrites par T.W. GREENE, *Protective Groups in Organic Syntheses,* A. Wiley-Interscience Publication (1981), ou par Mc OMIE, *Protective Groups in Organic Chemistry,* Plenum Press (1973).

A titre d'exemple, les groupements protecteurs peuvent être choisis parmi les radicaux triméthylsilyle, benzhydryle, tétrahydropyrannyle, formyle, acétyle, chloracétyle, trichloracétyle, trifluoracétyle, éthoxycarbonyle, tert-butoxycarbonyle, trichloroéthoxy-carbonyle, etc...

Une autre voie de synthèse permet d'aboutir à une seconde série de composés hydrophiles cationiques. Ce second mode de réalisation diffère du premier en ce que le squelette glucidique est transformé.

En effet, le produit de départ utilisé est un glycal de formule générale (II): 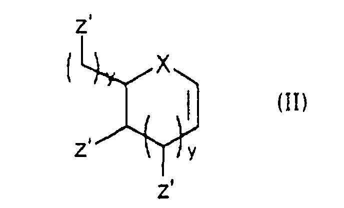 pour laquelle les substituants Z' sont des fonctions acétoxy. Les glycals utilisés sont disponibles commercialement ou bien sont obtenus par toute méthode connue de l'homme du métier à partir de glucides commerciaux, et notamment par réaction des acétobromosucres correspondants avec le couple zinc/cuivre.

Dans une première étape, le dit glycal acétylé est alkylé selon les méthodes classiques connues de l'homme du métier.

Notamment, on met en oeuvre la réaction de Ferrier par action, en présence d'un acide de Lewis, d'un aminoalcool, d'un alkyloxycarbonylalcool, d'un carboxyalcool, ou de tout autre alcool fonctionnalisé par un groupement permettant le couplage à une région lipophile.

De préférence, la réaction est conduite en présence de trifluorure de bore dans un solvant compatible, par exemple un éther comme l'éthyléther.

Dans une seconde étape, le glycoside insaturé obtenu est réduit selon les méthodes classiques connues de l'homme du métier.

Notamment, on effectue une réduction en milieu acide, par exemple par hydrogénation en milieu acide en présence de palladium sur charbon.

Puis, dans une troisième étape, les fonctions acétoxy présentes sur le cycle sont transformées en fonctions hydroxy par toute méthode connue de l'homme du métier.

Notamment, on opère par transestérification à l'aide d'un alcoolate, par exemple le méthanoate de sodium.

Dans une quatrième étape, les fonctions hydroxy présentes sur le cycle sont transformées en fonctions azide par toute méthode connue de l'homme du métier.

Notamment, on opère par exemple par action de l'acide hydrazoïque en présence de triphénylphosphine et de diéthylazodicarboxylate dans un solvant compatible avec la réaction.

Les solvants utilisables sont les solvants classiques d'azidation, et de préférence le tétrahydrofurane (THF).

On obtient ainsi une région hydrophile cationique de formule générale (III) : 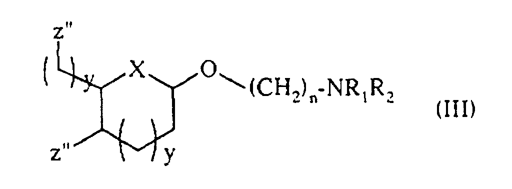 pour laquelle Z" est soit un atome d'hydrogène, soit un groupement azide, l'un au mois des Z" étant différent de l'hydrogène.

La partie lipophile peut être couplée sur la fonction amino -NR₁R₂ selon les méthodes connues de l'homme du métier, notamment par couplage peptidique (Bodanski M., *Principles and Practices of Peptides Synthesis,* Ed. Springe-Verlag), ou encore par condensation.

L'alcool mis en oeuvre au cours de la première étape comportant une fonction amino, ester ou tout autre fonction permettant le couplage à une région lipophile, il est préférable au préalable de la protéger. La protection et l'élimination du radical protecteur avant le couplage avec la région lipophile, s'effectuent selon les méthodes habituelles.

La protection peut être réalisée par tout groupement compatible et dont la mise en oeuvre et l'élimination n'altère pas le reste de la molécule. Notamment, on opère selon les méthodes décrites par T.W. GREENE, *Protective Groups in Organic Syntheses,* A. Wiley-Interscience Publication (1981), ou par Mc OMIE, *Protective Groups in Organic Chemistry,* Plenum Press (1973).

A titre d'exemple, les groupements protecteurs peuvent être choisis parmi les radicaux triméthylsilyle, benzhydryle, tétrahydropyrannyle, formyle, acétyle, chloracétyle, trichloracétyle, trifluoracétyle, éthoxycarbonyle, tert-butoxycarbonyle, trichloroéthoxy-carbonyle, phtalimido, etc...

Enfin, dans une dernière étape, les fonctions azides portées par le cycle à 5 ou 6 chaînons sont transformées en fonctions amino par les méthodes classiques connues de l'homme du métier qui n'altèrent pas le reste de la molécule. Préférentiellement, on opère par action de triphénylphosphine en présence d'eau.

Toute autre méthode connue de l'homme du métier conduisant aux agents de transfert d'acides nucléiques selon la présente invention entre également dans le cadre de l'invention.

A titre d'exemple non-limitatif, le premier mode de réalisation de l'invention conduit aux agents de transfert de formule (8a) et (8b), tandis que le second mode de réalisation conduit aux agents de transfert de formule (16) et (17), comme cela est précisé dans la partie "Exemples" de la présente description.

L'intérêt de cette seconde voie de synthèse de régions hydrophiles cationiques réside principalement dans le peu d'étapes nécessaires à la synthèse et dans la versatilité du synthon aminé obtenu. Une même tête cationique peut ainsi être condensée avec différents substituants hydrophobes.

Un autre objet de l'invention concerne une composition comprenant un agent de transfert d'acides nucléiques tel que défini précédemment et un acide nucléique. Préférentiellement, les compositions comprennent un rapport de 0,1 à 50 nanomoles de vecteur par µg d'acide nucléique. Avantageusement, ce rapport se situe entre 2 et 20 nanomoles de vecteur par µg d'acide nucléique et encore plus préférentiellement entre 4 et 12 nanomoles de vecteur par µg d'acide nucléique. Plus particulièrement l'agent de transfert et l'acide nucléiques sont présent dans un rapport de 8 à 12 nanomoles de vecteur par µg d'acide nucléique.

On entend au sens de l'invention par "acide nucléique" aussi bien un acide désoxyribonucléique qu'un acide ribonucléique. Il peut s'agir de séquences naturelles ou artificielles, et notamment d'ADN génomique (ADNg), d'ADN complémentaire (ADNc), d'ARN messager (ARNm), d'ARN de transfert (ARNt), d'ARN ribosomique (ARNr), de séquences hybrides ou de séquences synthétiques ou semi-synthétiques, d'oligonucléotides modifiés ou non. Ces acides nucléiques peuvent être d'origine humaine, animale, végétale, bactérienne, virale, etc... Ils peuvent être obtenus par toute technique connue de l'homme du métier, et notamment par criblage de banques, par synthèse chimique, ou encore par des méthodes mixtes incluant la modification chimique ou enzymatique de séquences obtenues par criblage de banques. Ils peuvent être modifiés chimiquement.

Concernant plus particulièrement les acides désoxyribonucléiques, ils peuvent être simple ou double brin de même que des oligonuléotides courts ou des séquences plus longues. En particulier, les acides nucléiques sont avantageusement constitués par des plasmides, des vecteurs, des épisomes, des cassettes d'expression, etc... Ces acides désoxyribonucléiques peuvent porter une origine de réplication fonctionnelle ou non dans la cellule cible, un ou plusieurs gènes marqueurs, des séquences régulatrices de la transcription ou de la réplication, des gênes d'intérêt thérapeutique, des séquences antisens modifiées ou non, des régions de liaison à d'autres composants cellulaires, etc...

De préférence, l'acide nucléique comprend une cassette d'expression constituée d'un ou plusieurs gènes d'intérêt sous contrôle d'un ou plusieurs promoteurs et d'un terminateur transcriptionnel actifs dans les cellules cibles.

Au sens de l'invention, on entend par gêne d'intérêt thérapeutique notamment tout gène codant pour un produit protéique ayant un effet thérapeutique. Le produit protéique ainsi codé peut être notamment une protéine ou un peptide. Ce produit protéique peut être exogène homologue ou endogène vis-à-vis de la cellule cible, c'est-à-dire un produit qui est normalement exprimé dans la cellule cible lorsque celle-ci ne présente aucune pathologie. Dans ce cas, l'expression d'une protéine permet par exemple de pallier une expression insuffisante dans la cellule ou l'expression d'une protéine inactive ou faiblement active en raison d'une modification, ou encore de surexprimer ladite protéine. Le gène d'intérêt thérapeutique peut aussi coder pour un mutant d'une protéine cellulaire, ayant une stabilité accrue, une activité modifiée, etc... Le produit protéique peut également être hétérologue vis-à-vis de la cellule cible. Dans ce cas, une protéine exprimée peut par exemple compléter ou apporter une activité déficiente dans la cellule, lui permettant de lutter contre une pathologie, ou stimuler une réponse immunitaire.

Parmi les produits thérapeutiques au sens de la présente invention, on peut citer plus particulièrement les enzymes, les dérivés sanguins, les hormones, les lymphokines : interleukines, interférons, TNF, etc... (FR 92/03120), les facteurs de croissance, les neurotransmetteurs ou leurs précurseurs ou enzymes de synthèse, les facteurs trophiques (BDNF, CNTF, NGF, IGF, GMF, aFGF, bFGF, NT3, NT5, HARP/pléiotrophine, etc...) les apolipoprotéines (ApoAl, ApoAIV, ApoE, etc..., FR 93/05125), la dystrophine ou une minidystrophine (FR 91/11947), la protéine CFTR associée à la mucoviscidose, les gènes suppresseurs de tumeurs (p53, Rb, Rap1A, DCC, k-rev, etc..., FR 93/04745), les gènes codant pour des facteurs impliqués dans la coagulation (Facteurs VII, VIII, IX), les gènes intervenant dans la réparation de l'ADN, les gènes suicides (thymidine kinase, cytosine déaminase), les gènes de l'hémoglobine ou d'autres transporteurs protéiques, les enzymes du métabolisme, catabolisme etc...

L'acide nucléique d'intérêt thérapeutique peut également être un gène ou une séquence antisens, dont l'expression dans la cellule cible permet de contrôler l'expression de gènes ou la transcription d'ARNm cellulaires. De telles séquences peuvent, par exemple, être transcrites dans la cellule cible en ARN complémentaires d'ARNm cellulaires et bloquer ainsi leur traduction en protéine, selon la technique décrite dans le brevet EP 140 308. Les gènes thérapeutiques comprenent également les séquences codant pour des ribozymes, qui sont capables de détruire sélectivement des ARN cibles (EP 321 201).

Comme indiqué plus haut, l'acide nucléique peut également comporter un ou plusieurs gènes codant pour un peptide antigénique, capable de générer chez l'homme ou l'animal une réponse immunitaire. Dans ce mode particulier de mise en oeuvre, l'invention permet la réalisation soit de vaccins soit de traitements immunothérapeutiques appliqués à l'homme ou à l'animal, notamment contre des microorganismes, des virus ou des cancers. Il peut s'agir notamment de peptides antigéniques spécifiques du virus d'Epstein Barr, du virus HIV, du virus de l'hépatite B (EP 185 573), du virus de la pseudo-rage, du "syncitia forming virus", d'autres virus ou encore de peptides antigéniques spécifiques de tumeurs (EP 259 212).

Préférentiellement, l'acide nucléique comprend également des séquences permettant l'expression du gêne d'intérêt thérapeutique et/ou du gêne codant pour le peptide antigénique dans la cellule ou l'organe désiré. Il peut s'agir des séquences qui sont naturellement responsables de l'expression du gène considéré lorsque ces séquences sont susceptibles de fonctionner dans la cellule infectée. Il peut également s'agir de séquences d'origine différente (responsables de l'expression d'autres protéines, ou même synthétiques). Notamment, il peut s'agir de séquences promotrices de gènes eucaryotes ou viraux. Par exemple, il peut s'agir de séquences promotrices issues du génome de la cellule que l'on désire infecter. De même, il peut s'agir de séquences promotrices issues du génome d'un virus. A cet égard, on peut citer par exemple les promoteurs des gènes E1A, MLP, CMV, RSV, etc... En outre, ces séquences d'expression peuvent être modifiées par addition de séquences d'activation, de régulation, etc... Il peut aussi s'agir de promoteur, inductible ou répressible.

Par ailleurs, l'acide nucléique peut également comporter, en particulier en amont du gêne d'intérêt thérapeutique, une séquence signal dirigeant le produit thérapeutique synthétisé dans les voies de sécrétion de la cellule cible. Cette séquence signal peut être la séquence signal naturelle du produit thérapeutique, mais il peut également s'agir de toute autre séquence signal fonctionnelle, ou d'une séquence signal artificielle. L'acide nucléique peut également comporter une séquence signal dirigeant le produit thérapeutique synthétisé vers un compartiment particulier de la cellule.

Les compositions de l'invention peuvent en outre comporter des adjuvants capables de s'associer aux complexes agents de transfert/acide nucléique et d'en améliorer le pouvoir transfectant. Dans un autre mode de mise en oeuvre, la présente invention concerne donc des compositions comprenant un acide nucléique, un agent de transfert tel que défini précédemment et un ou plusieurs adjuvants capables de s'associer aux complexes nucléolipidiques et d'en améliorer le pouvoir transfectant.

Dans cette optique, les compositions de l'invention peuvent comprendre comme adjuvant, un ou plusieurs lipides neutres. De telles compositions sont particulièrement avantageuses, notamment lorsque le rapport de charges agent de transfert/acide nucléique est faible. La demanderesse a en effet montré que l'addition d'un lipide neutre permet d'améliorer la formation des particules nucléolipidiques et de favoriser la pénétration de la particule dans la cellule en déstabilisant sa membrane.

Plus préférentiellement, les lipides neutres utilisés dans le cadre de la présente invention sont des lipides à deux chaînes grasses. De manière particulièrement avantageuse, on utilise des lipides naturels ou synthétiques, zwitterioniques ou dépourvus de charge ionique dans les conditions physiologique. Il peuvent être choisis plus particulièrement parmi la dioleoylphosphatidyléthanolamine (DOPE), l'oléoylpalmitoylphos-phatidyléthanolamine (POPE), les di-stéaroyl, -palmitoyl, -mirystoyl phosphatidyléthanolamines ainsi que leurs dérivé N-méthylés 1 à 3 fois, les phosphatidylglycérols, les diacylglycérols, les glycosyldiacylglycérols, les cérébrosides (tels que notamment les galactocérébrosides), les sphingolipides (tels que notamment les sphingomyélines) ou encore les asialogangliosides (tels que notamment les asialoGM1 et GM2).

Ces différents lipides peuvent être obtenus soit par synthèse, soit par extraction à partir d'organes (exemple : le cerveau) ou d'oeufs, par des techniques classiques bien connues de l'homme du métier. En particulier, l'extraction des lipides naturels peut être réalisée au moyen de solvants organiques (voir également Lehninger, Biochemistry).

Plus récemment, la demanderesse a démontré qu'il était également particulièrement avantageux d'employer à titre d'adjuvant, un composé intervenant ou non directement au niveau de la condensation dudit acide nucléique (WO 96/25508). La présence d'un tel composé, au sein d'une composition selon l'invention, permet de diminuer la quantité d'agent transfectant, avec les conséquences bénéfiques qui en découlent sur le plan toxicologique, sans porter un préjudice quelconque à l'activité transfectante. Par composé intervenant au niveau de la condensation de l'acide nucléique, on entend définir un composé compactant, directement ou non, l'acide nucléique. Plus précisément, ce composé peut soit agir directement au niveau de l'acide nucléique à transfecter soit intervenir au niveau d'un composé annexe qui lui est directement impliqué dans la condensation de cet acide nucléique. De préférence, il agit directement au niveau de l'acide nucléique. Par exemple, l'agent précompactant peut être tout polycation, par exemple la polylysine. Selon un mode de réalisation préféré, cet agent intervenant au niveau de la condensation de l'acide nucléique dérive en tout ou partie d'une protamine, d'une histone, ou d'une nucléoline et/ou de l'un de leurs dérivés. Un tel agent peut également être constitué, en tout ou partie, de motifs peptidiques (KTPKKAKKP) et/ou (ATPAKKAA), le nombre des motifs pouvant varier entre 2 et 10. Dans la structure du composé selon l'invention, ces motifs peuvent être répétés de manière continue ou non. C'est ainsi qu'ils peuvent être séparés par des liens de nature biochimique, par exemple par un ou plusieurs acides aminés, ou de nature chimique.

Dans un mode de réalisation particulièrement avantageux, les compositions de la présente invention comprennent en outre un élément de ciblage permettant d'orienter le transfert de l'acide nucléique. Cet élément de ciblage peut être un élément de ciblage extracellulaire permettant d'orienter le transfert de l'ADN vers certains, types cellulaires ou certains tissus souhaités (cellules tumorales, cellules hépatiques, cellules hématopoiétiques...). Il peut également s'agir d'un élément de ciblage intracellulaire permettant d'orienter le transfert de l'acide nucléique vers certains compartiments cellulaires privilégiés (mitochondries, noyau etc...). L'élément de ciblage peut être lié à l'agent de transfert d'acides nucléiques selon l'invention ou également à l'acide nucléique comme cela a été précisé précédemment.

Parmi les éléments de ciblage utilisables dans le cadre de l'invention, on peut citer les sucres, les peptides, les protéines, les oligonucléotides, les lipides, les neuromédiateurs, les hormones, les vitamines ou leurs dérivés. Préférentiellement, il s'agit de sucres de peptides ou de protéines tels que des anticorps ou des fragments d'anticorps, des ligands de récepteurs cellulaires ou des fragments de ceux-ci, des récepteurs ou des fragments de récepteurs, etc... En particulier, il peut s'agir de ligands de récepteurs de facteurs de croissance, de récepteurs de cytokines, de récepteurs de type lectines cellulaires, ou de ligands à séquence RGD avec une affinité pour les récepteurs de protéines d'adhésion comme les intégrines. On peut également citer les récepteurs de la transferrine, des HDL et des LDL, ou le transporteur du folate. L'élément de ciblage peut également être un sucre permettant de cibler des lectines tels que les récepteurs aux asialoglycoprotéines ou aux syalydés tel que le sialyde Lewis X, ou encore un fragment Fab d'anticorps, ou un anticorps simple chaîne (ScFv). Plus récemment, il a également été décrit des peptides ligands, naturels ou synthétiques, intéressants notamment pour leur sélectivité vis à vis de cellules spécifiques et capables de promouvoir efficacement l'internalisation au niveau de ces cellules. ( Bary et al. Nature Medicine, 2, 1996, 299-305).

L'association des éléments de ciblage aux complexes nucléolipidiques peut être effectuée par toute technique connue de l'homme du métier, par exemple par couplage à une partie hydrophobe ou à une partie qui interagit avec l'acide nucléique de l'agent de transfert selon l'invention, ou encore à un groupement qui interagit avec l'agent de transfert selon l'invention ou avec l'acide nucléique. Les interactions en question peuvent être, selon un mode préféré, de nature ionique ou covalente.

Selon une autre variante, les compositions de l'invention peuvent aussi éventuellement incorporer au moins un agent de surface non-ionique en quantité suffisante pour stabiliser la taille des particules de complexes nucléolipidiques. L'introduction d'agents de surface non-ionique prévient la formation d'agrégats, ce qui rend la composition plus particulièrement adaptée à une administration *in vivo.* Les compositions selon l'invention incorporant de tels agents de surface présentent un avantage sur le plan de l'innocuité. Elles présentent également un avantage supplémentaire en ce sens qu'elles diminuent le risque d'interférences avec d'autres protéines compte tenu de la réduction de la charge globale des compositions de complexes nucléolipidiques.

Les agents de surface sont constitués avantageusement d'au moins un segment hydrophobe, et d'au moins un segment hydrophile. Préférentiellement, Le segment hydrophobe est choisi parmi les chaînes aliphatiques, les polyoxyalkylène hydrophobes, les polyester d'alkylidène, les polyéthylène glycol à tête polyéther benzylique et le cholestérol, et le segment hydrophile est avantageusement choisi parmi les polyoxyalkylène hydrophiles, les alcools polyvinyliques, les polyvinylpyrrolidones ou les saccharides. De tels agents de surface non-ioniques ont été décrits dans la demande PCT/FR 98/00222.

La présente invention a également pour objet l'utilisation des agents de transfert décrits ci-avant pour le tranfert d'acides nucléiques (et plus généralement de polyanions) dans les cellules. Une telle utilisation est particulièrement avantageuse car ces agents transfectants augmentent l'efficacité de la transfection tout en présentant une toxicité cellulaire nulle ou très reduite.

Pour des utilisations *in vivo*, par exemple pour l'étude de la régulation de gènes, la création de modèles animaux de pathologies ou en thérapie, les compositions selon l'invention peuvent être formulées en vue d'administrations par voie topique, cutanée, orale, rectale, vaginale, parentérale, intranasale, intraveineuse, intramusculaire, sous-cutanée, intraoculaire, transdermique, intratrachéale, intrapéritonéale, etc... De préférence, les compositions pharmaceutiques de l'invention contiennent un véhicule pharmaceutiquement acceptable pour une formulation injectable, notamment pour une injection directe au niveau de l'organe désiré, ou pour une administration par voie topique (sur peau et/ou muqueuse). Il peut s'agir en particulier de solutions stériles, isotoniques, ou de compositions sèches, notamment lyophilisées, qui, par addition selon le cas d'eau stérilisée ou de sérum physiologique, permettent la constitution de solutés injectables. Les doses d'acide nucléique utilisées pour l'injection ainsi que le nombre d'administrations peuvent être adaptées en fonction de différents paramètres, et notamment en fonction du mode d'administration utilisé, de la pathologie concernée, du gène à exprimer, ou encore de la durée du traitement recherchée. En ce qui concerne plus particulièrement le mode d'administration, il peut s'agir soit d'une injection directe dans les tissus, par exemple au niveau des tumeurs, ou les voies circulatoires, soit d'un traitement de cellules en culture suivi de leur réimplantation *in vivo,* par injection ou greffe. Les tissus concernés dans le cadre de la présente invention sont par exemple les muscles, la peau, le cerveau, les poumons, le foie, la rate, la moelle osseuse, le thymus, le coeur, la lymphe, le sang, les os, les cartilages, le pancréas, les reins, la vessie, l'estomac, les intestins, les testicules, les ovaires, le rectum, le système nerveux, les yeux, les glandes, les tissus conjonctifs, etc...

L'invention concerne en outre une méthode de transfert d'acides nucléiques dans les cellules comprenant les étapes suivantes
(1) la mise en contact de l'acide nucléique avec un agent de transfert tel que défini ci-avant, pour former un complexe nucléolipidique, et
(2) la mise en contact des cellules avec le complexe formé en (1).

La mise en contact des cellules avec le complexe peut être réalisée par incubation des cellules avec ledit complexe nucléolipidique (pour des utilisations *in vitro* ou *ex vivo),* ou par injection du complexe dans un organisme (pour des utilisations *in vivo).* L'incubation est réalisée de préférence en présence de par exemple de 0,01 à 1000 µg d'acide nucléique pour 10⁶ cellules. Pour une administration *in vivo,* des doses d'acide nucléique comprises entre 0,01 et 10 mg peuvent par exemple être utilisées.

Dans le cas où les compositions de l'invention contiennent en outre un ou plusieurs adjuvants tels que définis précédemment, le ou les adjuvants sont préalablement mélangés au lipide selon l'invention ou à l'acide nucléique.

La présente invention fournit ainsi une méthode particulièrement avantageuse pour le transfert d'acides nucléiques, notamment pour le traitement de maladies, comprenant l'administration *in viv*o, *ex vivo* ou *in vitro* d'un acide nucléique codant pour une protéine ou pouvant être transcrit en un acide nucléique apte à corriger ladite maladie, ledit acide nucléique étant associé à un composé de formule générale (I) dans les conditions définies ci-avant. Plus particulièrement, cette méthode est applicable aux maladies résultant d'une déficience en un produit protéique ou nucléique, l'acide nucléique administré codant pour ledit produit protéique ou étant trranscrit en un produit nucléique ou encore constituant ledit produit nucléique.

L'invention s'étend à toute utilisation d'un agent de transfert d'acides nucléiques selon l'invention pour la transfection de cellules. Egalement, l'invention s'étend à toute utilisation d'un agent transfectant tel que défini ci-avant pour la fabrication d'un médicament contenant au moins un acide nucléique à transfecter.

Les agents de transfert d'acides nucléiques de l'invention sont particulièrement utilisables pour le transfert d'acides nucléiques dans des cellules primaires ou dans des lignées établies. Il peut s'agir de cellules fibroblastiques, musculaires, nerveuses (neurones, astrocytes, cellules gliales), hépatiques, de la lignée hématopoiétique (lymphocytes, CD34, dendritiques, etc...), épithéliales etc..., sous forme différenciées ou pluripotentes (précurseurs).

Outre les dispositions qui précèdent, la présente invention comprend également d'autres caractéristiques et avantages qui ressortiront des exemples et figures qui suivent, et qui doivent être considérés comme illustrant l'invention sans en limiter la portée.

### LISTE DES FIGURES

Figure 1/5 : Schéma de synthèse des composés (1) à (5).

Figure 2/5 : Schéma de synthèse des composés (5) à (8a) et (8b).

Figure 3/5 : Schéma de synthèse des composés (9) à (16).

Figure 4/5 : Mesure de l'efficacité de la transfection *in vitro* des composés (8a) et (8b) par l'intermédiaire de la mesure de l'activité luciférase dans les cellules HeLa, en l'absence de sérum. Les mesures ont été faites à différents rapports nanomoles de vecteur/µg d'ADN, et sont représentées par les barres. Le dosage de protéine, pour chacune des compositions, a également été fait et est représenté par une courbe en ligne pleine sur le même graphique.

Figure 5/5: Mesure de l'efficacité de la transfection *in vitro* des composés (8a) et (8b) par l'intermédiaire de la mesure de l'activité luciférase dans les cellules NIH 3T3, en l'absence de sérum. Les mesures ont été faites à différents rapports nanomoles de vecteur/µg d'ADN, et sont représentées par les barres.

### MATERIELS ET METHODES

- Les purifications sur HPLC (High Performance Liquid Chromatography) ont été effectuées avec un appareil Waters LC 4000, sur une colonne Vydac C4 éluée par un gradient d'acétonitrile dans l'eau.
- Les mesures de fluorescence ont été réalisées sur Perkin Elmer LS50B, en utilisant des longueurs d'onde d'excitation et d'émission respectivement de 260 nm et 590 nm. Les largeurs de fentes pour l'excitation et l'émission sont réglées à 5 nm. La valeur de fluorescence est enregistrée après ajout de 5 µg de bromure d'éthidium/ml en concentration finale.
- Les spectres de résonance magnétique nucléaire (RMN) ont été enregistrés, à 300,13 MHz pour le proton ou à 75,47 MHz pour le carbone, sur un appareil Bruker MSL 300 dans le chloroforme deutérié.

### EXEMPLES

### EXEMPLE 1: Synthèse de l'agent de transfert 8(a) à partir du méthylbenzyloxy-6-galactopyranose.

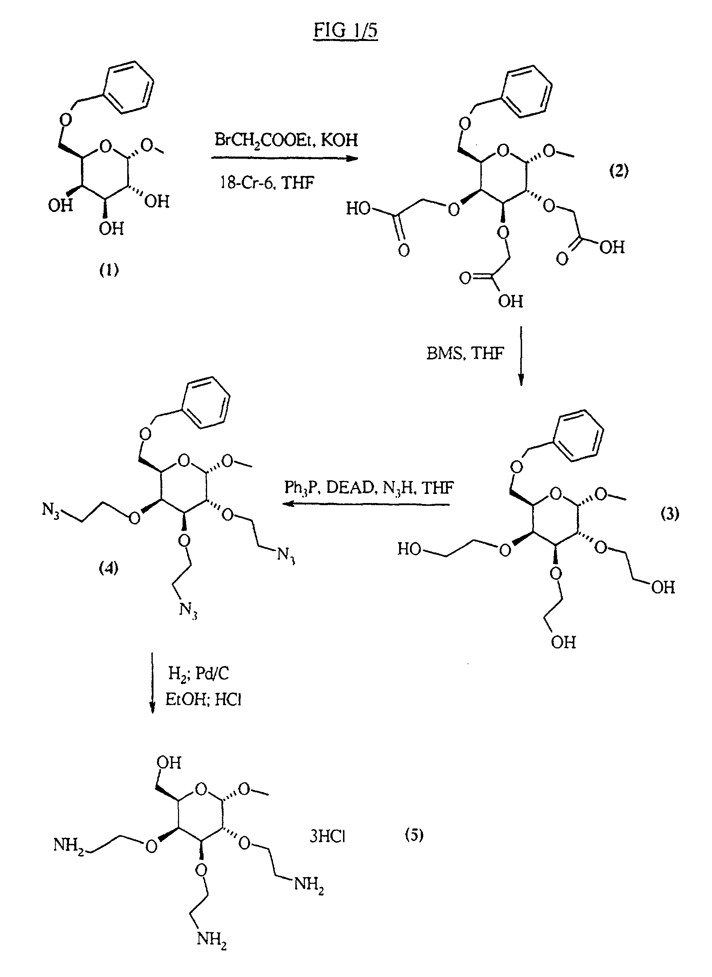

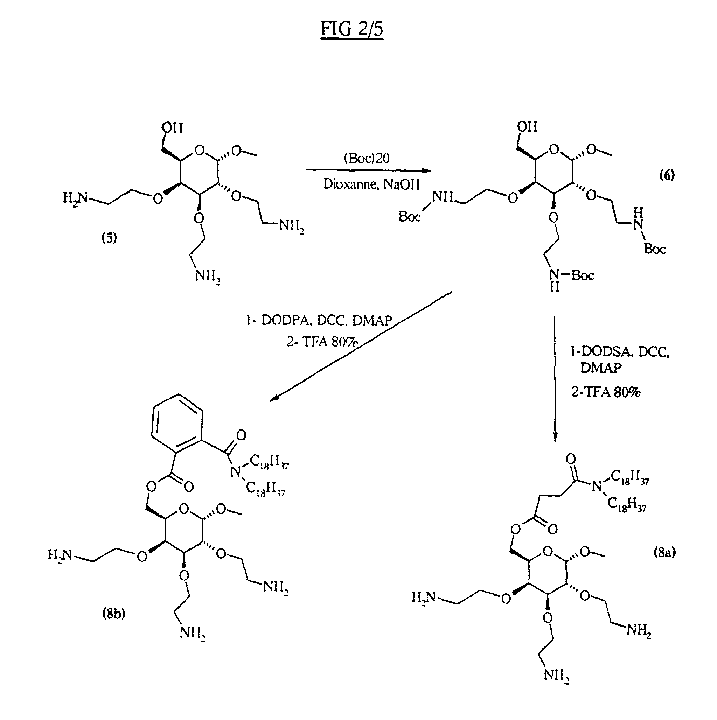

Les différentes étapes réactionnelles sont schématisées aux figures 1/5 et 2/5.

### a) Synthèse du méthyl-benzyloxy-6-tri-O-(carboxy-2'-éthyle)-2,3,4-β-D-galactopyranoside (2)

28,4 g (0,1 mol) de méthyl-benzyloxy-6-b-D-galactopyranoside (1) sont dissous dans 400 ml de THF et placés à 0°C sous agitation. On ajoute 32 g (5,7 équivalents) de potasse finement broyée, 0,5 g (2%) d'éther couronne 18-Cr-6 et 32,5 ml (1,5 équivalents) de bromoacétate d'éthyle. Après deux heures, on évapore à sec, on redissout dans l'eau et on neutralise la solution obtenue avec de l'acide chlorhydrique 2N. On obtient ainsi 32 g de produit (2) (rendement : 70%) qui est extrait par du dichlorométhane.
RMN ¹³C: δ, 172,94; 136,87; 127,93; 127,41; 98,89; 97,22; 78,74; 76,17; 72,97; 69,52; 67,93; 65,39; 54,75.

### b) Synthèse du méthyl-benzyloxy-6-tri-O-(hydroxy-2'-éthyle)-2,3,4-β-D-galactopyranoside (3)

30 g (0,065 mol) du triacide précédent (2) sont dissous dans 400 ml de THF et placés à 0°C sous azote. On ajoute goutte à goutte tout en agitant 40 ml de borane-diméthyle sulfure. On agite encore deux heures à température ambiante puis on ajoute avec précaution du méthanol en excès. On évapore ensuite la solution à sec et on redissout le solide obtenu plusieurs fois dans du méthanol. On obtient ainsi 24,5 g de produit final (3) (rendement : 90%) qui est purifié sur colonne de silice éluée par de l'acétate d'éthyle.

### c) Synthèse du méthyl-benzyloxy-6-tri-O-(azido-2'-éthyle)-2,3,4-β-D-galactopyranoside (4)

20 g (0,048 mol) de triol (3) obtenus à l'étape précédente sont dissous dans 500 ml de THF. On ajoute 40 g (3,2 équivalents) de triphénylphosphine, 160 ml (3,7 équivalents) d'une solution d'acide hydrazoïque 1,1 M dans le toluène, puis 24,2 ml (3,2 équivalents) de diéthylazodicarboxylate. Après une heure, la solution est évaporée et purifiée sur une colonne de silicagel éluée par un mélange heptane/acétate d'éthyle (6:4). On obtient ainsi 17,6 g de produit (4) (rendement : 90%).
RMN ¹³C: δ, 128,47; 127,80; 98,23; 79,05; 76,27; 73,55; 71,92; 70,15; 69,95; 68,77; 68,14; 55,37; 51,41; 51,11; 50,87.

### d) Synthèse du méthyl-tri-O-(amino-2'-éthyl)-2,3,4-β-D-galactopyranoside, trischlorhydrate (5)

15 g (0,03 mol) de triazide (4) sont mis en solution dans 200 ml d'éthanol. On ajoute 7,5 ml d'acide chlorhydrique 12 N, puis on traite par l'hydrogène en présence de palladium sur carbone pendant 3 heures. Après filtration, le solvant est évaporé et le résidu (5) obtenu (12,6 g, rendement : 95%) est utilisé directement dans l'étape suivante.

### e) Synthèse du méthyl-tri-O-(tert-butyl-carbamido-2'-éthyl)-2,3,4-β-D-galactopyranoside (6)

12 g (0,028 mol) de chlorhydrate (5) sont mis en solution dans un mélange de 100 ml de dioxanne et 85 ml d'hydroxide de sodium 1 N. On ajoute 18,7 g (3,3 équivalents) de di-tert-butyl dicarbonate et on agite à température ambiante pendant deux heures. Le mélange réactionnel est évaporé puis extrait par du dichlorométhane (3 fois 50 ml). Les phases organiques sont combinées puis séchées sur sulfate de sodium et évaporées. On obtient ainsi 15,56 g (rendement : 90%) de résidu (6) purifié sur colonne de silicagel élué par de l'acétate d'éthyle.

### f) Synthèse du méthyl-dioctadécylamido-succinyl-6-tri-O-(tert-butyl-carbamido-2'-éthyl)-2,3,4-β-D-gaIactopyranoside (7a)

10 g (0,016 mol) du produit précédent (6) sont dissous dans 300 ml de dichlorométhane. On ajoute successivement 14,6 g (1,5 équivalents) d'acide dioctadécylamidosuccinique, 1,95 g (1 équivalent) de diméthylaminopyridine et 6,5 g (2 équivalents) de dicyclohexylcarbodiimide. L'acide dioctadécylamidosuccinique est obtenu par réaction de la dioctadécylamine et de l'anhydride succinique. Après une nuit, on ajoute avec précaution 10 ml (1,7 g) d'une solution d'acide oxalique dans le méthanol et on laisse agir une heure (dégagement de monoxyde de carbone). On filtre la solution, on l'évaporé à sec et on purifie le produit obtenu sur colonne de silicagel éluée par une solution heptane/acétate d'éthyle (7:3). On obtient ainsi 12,8 g de produit (7a) (rendement : 65%).

### g) Synthèse du méthyl-dioctadécylamido-succinyl-6-tri-O-(amino-2'-éthyl)-2,3,4-β-D-galactopyranoside, tris trifluoroacétate (8a)

10 g (0,008 mol) de produit (7a) sont dissous dans 20 ml d'acide trifluoroacétique à 90%. Après une demi-heure, on évapore la solution et on reprend par 3 fois 20 ml de toluène. Le résidu (8a) obtenu est purifié par HPLC (RP4; eau/acétonitrile; 0->100%; 20 minutes). On obtient ainsi 9,7 g de produit (8a) (rendement : 95%).
MH⁺ =1304.

### EXEMPLE 2: Synthèse du composé 8(b) à partir du méthyl-benzyloxy-6-galactopyranose.

Les différentes étapes réactionnelles sont schématisées aux figures 1/5 et 2/5.

Les premières phases de synthèse sont identiques aux étapes de synthèse a) à e) décrites dans l'exemple 1. Puis, on continue de la façon suivante :

### f) Synthèse du méthyl-dioctadécylamido-phtalyl-6-tri-O-(tert-butyl-carbamido-2'-éthyl)-2,3,4-β-D-galactopyranoside (7b)

Ce produit est obtenu de la même manière que le produit (7a) mais en utilisant l'acide dioctadécylamidophtalique à la place de l'acide dioctadécylamidosuccinique.

### g) Synthèse du méthyl-dioctadécylamido-phtalyl-6-tri-O-(amino-2'-éthyl)-2,3,4-β-D-galactopyranoside, tris trifluoroacétate (8b)

On obtient le composé (8b) à partir du produit (7b) obtenu à l'étape précédente, de la même manière que le produit (8a).
MH⁺= 1317.

### EXEMPLE 3: Synthèse de l'agent de transfert (16) à partir du triacétyl glycal

### a) Synthèse du phtalimidopropyl-di-O-acétyl-4,6-bisdesoxy-2,3-b-D-erythrohexèno-2-pyranoside (10)

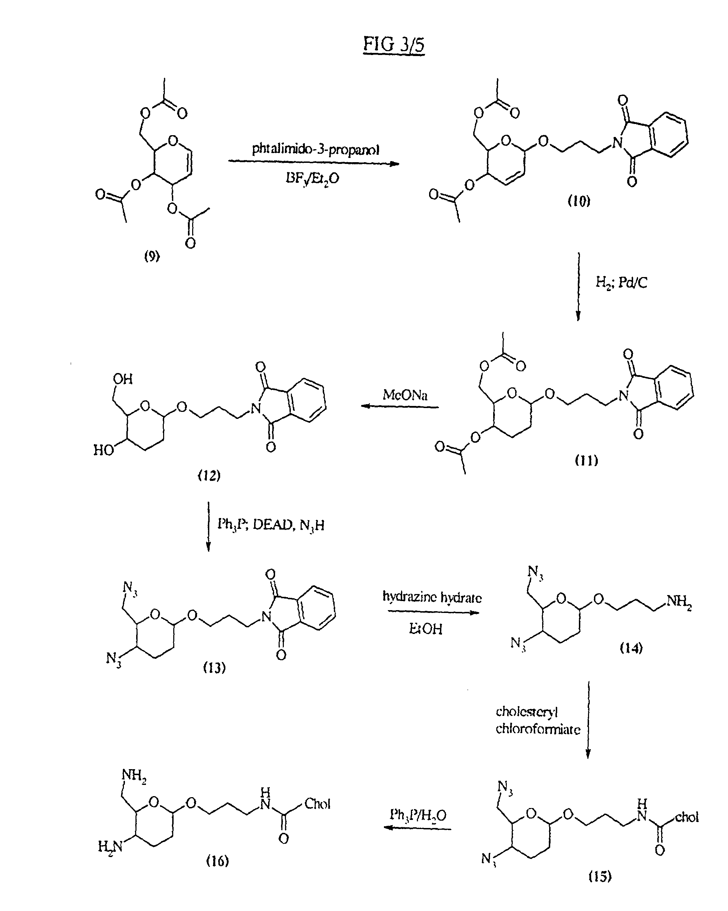

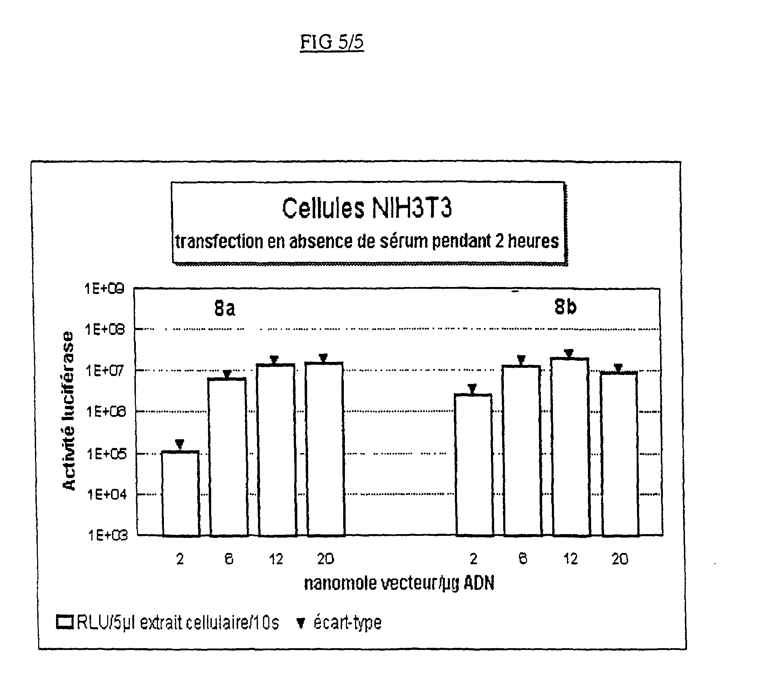

Les différentes étapes réactionnelles sont représentées à la figure 3/5.

13,17 g (0,048 mol) de triacétyl-D-glucal (9) sont dissous dans 250 ml de dichlorométhane. On ajoute 11 g (1,1 équivalents) de phtalimidopropanol et 1,56 ml (0,26 équivalents) d'étherate de trifluorure de bore. Après une heure à température ambiante, on neutralise le mélange par une solution saturée de bicarbonate de sodium, on décante, on sèche et on évapore la solution. On obtient ainsi 16 g de produit (10) (rendement : 80%) purifié par chromatographie sur colonne de silicagel éluée par une solution heptane/acétate d'éthyle (5:5).

### b) Synthèse du phtalimidopropyl-di-O-acétyl-4,6-bisdesoxy-2,3-β-D-glucopyranoside (11)

16 g (0,038 mol) de glycoside (10) sont dissous dans 200 ml d'éthanol. On ajoute 0,16 g de palladium à 10% sur carbone, et on traite par l'hydrogène pendant deux heures.

Après filtration, la solution est évaporée. On obtient ainsi 15,78 g de produit (11) (rendement : 99%).

### c) Synthèse du phtalimidopropyl-bisdesoxy-2,3-β-D-glucopyranoside (12)

15 g (0,036 mol) de produit précédent (11) sont dissous dans 250 ml de méthanol, puis on ajoute 3,5 ml (0,1 équivalents) de méthylate de sodium 1 N. Après deux heures, on neutralise par de la résine amberlite IR120. On obtient après filtration et évaporation 11,5 g de produit (12) (rendement : 96%).

### d) Synthèse du phtalimidopropyl-diazido-4,6-tétra-desoxy-2,3,4,6-β-D-glucopyranoside (13)

11 g (0,033 mol) de produit désacétylé (12) sont dissous dans 250 ml de THF. Tout en agitant, on ajoute successivement 21,5 g (2,5 équivalents) de triphénylphosphine et 110,5 ml (3,7 équivalents) d'acide hydrazoïque 1,1 M dans le toluène. 12,88 ml (2,5 équivalents) de diéthylazodicarboxylate sont ajoutés à une vitesse telle que la température du mélange ne dépasse pas 30°C. Après une heure, la solution est évaporée à sec, et le produit obtenu est purifié sur colonne de silicagel éluée par un mélange heptane/acétate d'éthyle (8:2). On obtient ainsi 7,2 g de produit (13) (rendement : 57%).

### e) Synthèse de l'aminopropyl-diazido-4,6-tétra-desoxy-2,3,4,6-β-D-glucopyranoside (14)

7 g (0,018 mol) de produit (13) sont dissous dans 500 ml d'éthanol, puis on ajoute 2,6 ml dl'hydrate d'hydrazine, et on agite à 40°C pendant deux heures. Après évaporation, on redissout dans du dichlorométhane et on laisse cristalliser le phtalylhydrazide qui s'est formé. On filtre et on évapore la solution. On obtient ainsi 4,17 g de produit (14) (rendement : 90%).

### f) Synthèse du (cholestéryl-O-formamidopropyl)-diazido-4,6-tétra-desoxy-2,3,4,6-β-D-glucopyranoside (15)

6,34 g (0,9 équivalents) de cholestéryl chloroformiate et 4 g (0,016 mol) de produit (14) sont mis en solution dans 200 ml d'ether éthylique. Un précipité se forme aussitôt, et on ajoute alors 50 ml de soude 1 N. Après quelques minutes, la solution redevient limpide. On laisse encore 30 minutes sous agitation, puis on décante, on lave avec 2 fois 50 ml d'eau, et on évapore à sec la solution. On obtient ainsi 9,4 g de produit (15) purifié sur colonne éluée par une solution heptane/acétate d'éthyle (6:4). (rendement : 90%).

### g) Synthèse du (cholestéryl-O-formamidopropyl)-diamino-4,6-tétra-desoxy-2,3,4,6-β-D-glucopyranoside (16)

9 g (0,013 mol) de diazide (15) sont dissous dans 150 ml de THF à 80%, puis on ajoute 14,2 g (4 équivalents) de triphényle phosphine et on chauffe à 50°C pendant une heure. Après refroidissement, on porte sur une colonne de résine anionique IRC-50 COOH, et on lave avec du THF à 80%. Le produit est ensuite élué sous forme d'acétate par le mélange THF/eau/acide acétique (64:16:10). On obtient ainsi 8,9 g de produit (16) (rendement : 90%).
RMN ¹³C: δ, 176,55; 156.65; 139,84; 132,02; 128,46; 125,46; 122,44; 97,11; 74,28; 56,66; 56,12; 50,02; 43,70; 42,30; 39,51; 38,57; 36,98; 36,55; 36,16; 35,76; 31,87; 30,30; 29,89; 28,18; 23,82; 22,78; 22,53; 21,03; 19,31; 18,70; 11,84.

### EXEMPLE 4 : Synthèse du composé (17)

### a) Synthèse du benzyl-(5-O-acétyl-6-acétoxyméthyl-5,6-dihydro-2H-pyran-2-yloxy)-acétate

2,8 g de triacétyl D-glucal ( 0,01 mol) et 1,6 ml de benzylglycolate (1,1 équivalents) sont mis en solution dans 80 ml de chloroforme. On refroidit dans un bain de glace et on ajoute 0,651 ml d'étherate de trifluorure de bore (0,5 équivalents). Après 45 minutes, on neutralise avec une solution aqueuse saturée de bicarbonate de sodium, on sèche sur sulfate de sodium, on filtre et on concentre. On purifie par chromatographie sur colonne de silicagel, éluée par un mélange heptane/acétate d'éthyle (6:4). Le rendement est de 96%.

### b) Synthèse d l'acide (5-O-acétyl-6-acétoxyméthyl-tétrahydro-pyran-2-yloxy)-acétique :

3,5 g du produit précédent (0,0093 mol) sont hydrogénés à pression atmosphérique et à température ambiante, en présence 0,35 g de palladium à 10% sur carbone pendant 10 heures. On filtre sur célite, on concentre et on purifie par chromatographie sur colonne de silicagel, éluée par de l'acétate d'éthyle. Le rendement est de 90%.

### c) Synthèse du N,N-dioctadécyl- (5-O-acétyl-6-acétoxyméthyl-tétrahydro-pyran-2-yloxy)- acétamide :

0,675 g du produit précédent (0,0017 mol) sont dissous dans 8,6 ml de chloroforme. On ajoute 0,9 ml de diisopropyléthylamine, 0,899 g de dioctadécylamine (1 équivalent) et 0,426 g de dicyclohexyle carbodiimide (1,2 équivalents). Après une nuit à température ambiante, on concentre et on extrait le produit par de l'heptane. On purifie par chromatographie sur colonne de silicagel, éluée par un mélange heptane/acétate d'éthyle (8:2). Le rendement est de 70%.

### d) Synthèse de N,N-dioctadécyl- (5-hydroxy-6-hydroxyméthyl-tétrahydro-pyran-2-yloxy)-acétamide :

1,57 g du produit précédent (0,00198 mol) sont mis en solution dans 10 ml de méthanol. On ajoute 0,197 ml de méthylate de sodium (0,2 équivalents; méthanol 2M). Après deux heures à température ambiante, on neutralise et on concentre. On purifie par chromatographie sur colonne de silicagel, éluée par un mélange heptane/acétate d'éthyle (8:2). Le rendement est de 92%.

### e) Synthèse de N,N-dioctadécyl-(5-azido-6-azidométhyl-tétrahydro-pyran-2-yloxy)- acétamide (17) :

0,6 g de produit précédent (0,00087 mol) sont dissous dans 15 ml de THF. On ajoute 0,48 g de triphénylphosphine (2,1 équivalents) puis 0,288 ml de DEAD (2,1 équivalents). Quand la réaction exothermique est terminée, on ajoute 1,588 ml d'une solution d'acide hydrazoïque 1,38 M dans le toluène (2,5 équivalents). A la fin de la réaction on concentre, on reprend par de l'heptane, on filtre et on concentre. Puis, on purifie par chromatographie sur colonne de silicagel, éluée par un mélange heptane/acétate d'éthyle (8:2). Le rendement est de 72%.
RMN ¹¹C :δ, 173,21; 172,07; 162,22; 161,77; 118,34; 114,49; 110,65; 108,34; 96,88; 65,18; 64,50; 48,27; 46,54; 40,69; 35,86; 31,78; 30,94; 28,63; 27,56; 26,92; 22,54; 21,84; 13,94.

### EXEMPLE 5 : Association des agents transfectants de l'invention 8(a) et 8(b) avec l'ADN.

Cet exemple illustre la nature de l'association entre les agents transfectants de l'invention et l'ADN, ainsi que la formation de complexes.

Les agents transfectants selon l'invention ont été mis en solution dans l'eau à 10 mM. Les solutions ainsi obtenues sont transparentes et correspondent à une organisation sous forme de micelles de lipide cationique.

Les complexes agent tranfectant/ADN ont été réalisés suivant un rapport de 6 nmoles d'agent transfectant/µg d'ADN.

L'association de l'agent tranfectant 8(b) avec l'ADN procure un complexe de taille égale à 92 nm ± 27 nm, et une fluorescence de 7% dans l'eau et de 60% dans le chlorure de sodium NaCl à 300 mM.

L'agent transfectant de l'invention 8(a), associé avec l'ADN, permet la formation d'un complexe de taille voisine de 98 nm ± 20 nm, et une fluorescence de 6% dans l'eau et 55% dans le chlorure de sodium NaCl à 300 mM.

Ces résultats montrent que les agents transfectants selon l'invention sont capables de s'associer avec de l'ADN et de former des particules dont le diamètre moyen est d'environ de 100 nm, ce qui constitue une taille particulièrement adaptée au passage des membranes cellulaires. Cette association reste relativement stable lorsqu'on augmente la force ionique du milieu. En effet, à 300 mM, plus de 50 % de l'ADN reste associé avec les agents tranfectant de l'invention.

### EXEMPLE 6 : Transfection d'ADN in vitro avec les agents transfectants de l'invention 8(a) et 8(b)

L'efficacité de la transfection a été évaluée *in vitro* à différentes doses de vecteurs/µg d'ADN.

Le matériel génétique utilisé pour ces expériences est une construction de plasmide pCMV-Luc comportant le gène rapporteur "luciférase", dérivée du plasmide pGL2-basic Vector [Promega] par insertion d'un fragment Mlu I-Hind III contenant le promoteur du Cytomegalovirus humain (CMV) extrait du plasmide pcDNA3 [Invitrogen].

Des solutions d'acide nucléique diluées à 40 µg/ml dans du sérum physiologique (chlorure de sodium NaCl 0,15 M) ont également été utilisées.

Les produits décrits dans l'invention sont mis en solution dans l'eau à concentration variant de 80 µM à 800 µM, et mélangés volume à volume avec la solution d'ADN. La concentration saline finale est de 75 mM afin de préparer extemporanément des solutions cytofectantes.

Pour la transfection, les cellules sont cultivées dans les conditions appropriées en microplaques de 24 puits (2 cm²/puits) et sont transfectées alors qu'elles sont en phase exponentielle de croissance et à 50-70 % de la confluence.

Les cellules sont lavées par 2 fois 500 µl de milieu dépourvu de protéines sériques et remise en croissance en milieu sans sérum. 50 µl de mélange cytofectant [1µg d'ADN/puits] sont ajoutés aux cellules [3 puits/condition vecteur-ADN]. Le milieu de croissance est supplémenté par la quantité appropriée de sérum 2 heures après la transfection.

L'efficacité de transfection est évaluée à 48 heures post-transfection par une mesure de l'expression de la luciférase selon les recommandations données pour l'utilisation du kit Promega [Luciferase Assay System]. La toxicité des mélanges cytofectants est estimée par une mesure des concentrations protéiques dans les lysats cellulaires.

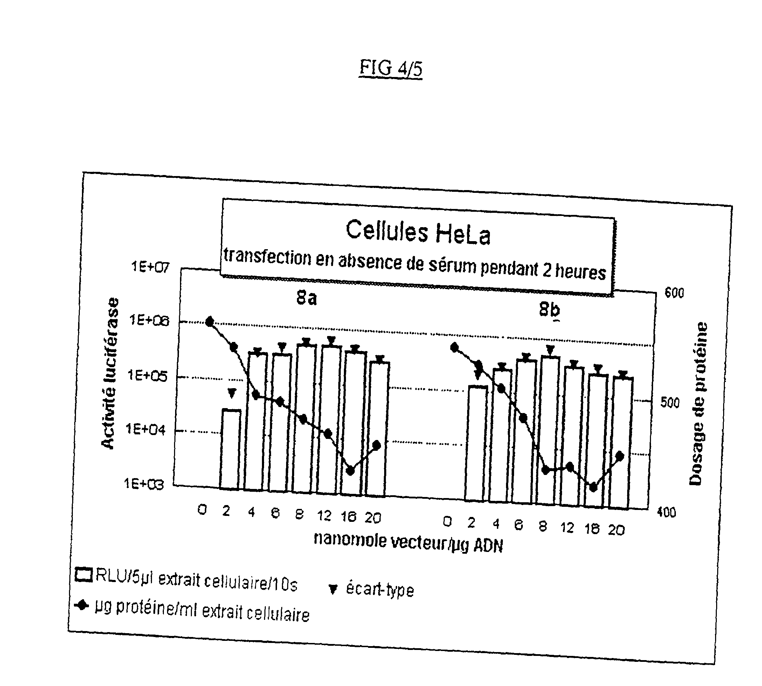

Les résultats obtenus pour la transfection des cellules HeLa sont rassemblés dans la figure 4/5, et ceux obtenus sur les cellules NIH3T3 sont indiqués dans la figure 5/5.

De ces deux figures ainsi que des résultats obtenus pour des rapports vecteurs/ADN supérieurs à 20 nmol/µg d'ADN (non montrés), il est possible de déduire que la transfection est particulièrement efficace lorsque le rapport vecteur/ADN est compris entre 2 et 20 nanomoles de vecteur par µg d'ADN.

Les résultats présentés montrent que pour les deux types cellulaires utilisés, l'efficacité de transfection est optimale pour un rapport compris entre 4 et 12 nanomoles de vecteur par µg d'ADN, et plus précisément entre 8 et 12 nanomoles de vecteur par µg d'ADN.

Par ailleurs, on constate que les histogrammes rendant compte de l'effet dose des vecteurs sont superposables pour les produits 8(a) et 8(b) quel que soit le type cellulaire considéré.

Les deux produits de l'invention ne sont pas toxiques aux doses de vecteur étudiées pour les cellules NIH 3T3. Nous n'observons en effet qu'une perte maximum de 20% des protéines cellulaires pour des doses supérieures à 8 nanomoles de vecteur par échantillon de cellules transfectées.

## Revendications

1. Agent transfectant comprenant au moins une région hydrophile cationique couplée à une région lipophile **caractérisé en ce que** ladite région hydrophile cationique a pour formule générale : 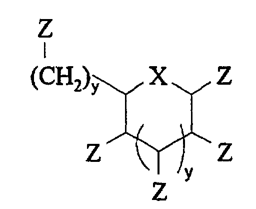 dans laquelle :
- y est un nombre entier égal à 0 ou 1, les différents y étant indépendants les uns des autres,
- X représente un atome d'oxygène, d'azote, de soufre ou de sélénium,
- les groupements Z représentent, indépendamment les uns des autres,
• un atome d'hydrogène,
• un groupement OR dans lequel R représente un atome d'hydrogène, un groupement méthyle, ou un groupement (CH2)ₙ-NR₁R₂ dans lequel n est un nombre entier choisi de 1 à 6 inclus et R₁ et R₂ représentent, indépendamment l'un de l'autre, un atome d'hydrogène ou un groupement (CH₂)q-NH₂, q pouvant varier de 1 à 6 inclus, les différents q étant indépendants les uns des autres,
• un groupement (CH₂)ₘ-NR₁R₂, dans lequel m est un nombre entier choisi de 0 à 6 inclus et R₁ et R₂ sont définis comme précédemment, ou bien
• un groupement "spacer" permettant la liaison de la région hydrophile cationique à la région lipophile,
étant entendu que l'un au moins des substituants Z est un groupement « spacer » et que au moins deux des substituants Z portent un groupement amino.

2. Agent transfectant selon la revendication 1 **caractérisé en ce que** dans la formule générale définissant la région hydrophile cationique, n est un nombre entier choisi parmi 2, 3 et 4 dans le groupement (CH2)ₙ-NR₁R₂.

3. Agent transfectant selon la revendication 1 **caractérisé en ce que** la formule générale définissant la région hydrophile cationique est un glycoside de forme pyranose ou furanose.

4. Agent transfectant selon la revendication 1 **caractérisé en ce que** dans la formule générale définissant la région hydrophile cationique, y est égal à 1 et X est un atome d'oxygène.

5. Agent transfectant selon la revendication 1 **caractérisé en ce que** ladite région lipophile est constituée d'une ou plusieurs chaînes aliphatiques linéaires ou ramifiées, saturées ou non et éventuellement halogénées, et/ou par un dérivé de stéroïde.

6. Agent transfectant selon la revendication 5 **caractérisé en ce que** les dites chaînes aliphatiques contiennent 10 à 22 atomes de carbone, et notamment 14, 16, 17, 18 et/ou 19 atomes de carbone.

7. Agent transfectant selon les revendications 5 et 6 **caractérisé en ce que** les dites chaînes aliphatiques sont choisies parmi (CH₂)₁₃CH₃, (CH₂)₁₅CH₃, (CH₂)₁₆CH₃, (CH₂)₁₇CH₃, et (CH₂)₁₈CH₃.

8. Agent transfectant selon la revendication 5 **caractérisé en ce que** le dérivé de stéroïde est choisi parmi le cholestérol, le cholestano1, le 3-α-5-cyclo-5-α-cholestan-6-β-ol, l'acide cholique, le cholestérylformiate, le cholestanylformiate, le 3α,5-cyclo-5α-cholestan-6β-yl formiate, la cholestérylamine, la 6-(1,5-diméthylhexyl)-3a,5a-diméthylhexadécahydro-cyclopenta[a]cyclopropa[2,3]cyclopenta[1,2-f]naphta-lèn-10-ylamine, ou la cholestanylamine.

9. Agent transfectant selon la revendication 1 **caractérisée en ce que** le groupement "spacer" permettant la liaison de la région hydrophile cationique à la région lipophile est constitué d'un groupe acide ou aminé comportant des fonctions hydrolysables.

10. Agent transfectant selon la revendication 9 **caractérisé en ce que** le groupement "spacer" est constitué d'une chaîne aliphatique ou aromatique et comporte un ou plusieurs groupements choisi parmi les amides, les esters, les éthers, les carbamates et les cycles aromatiques.

11. Agent transfectant selon la revendication 9 **caractérisé en ce que** le "spacer" est choisi parmi -O-CO-(CH₂)ₓ-COOH, -O-(CH₂)ₓ-COOH, -O-CO-(CH₂)ₓ-NH₂, -O-(CH₂)ₓ-NH₂, -NH-(CH₂)ₓ-NH₂, avec x représentant un entier compris entre 1 et 6 inclus.

12. Agent transfectant selon la revendication 1 **caractérisé en ce qu'**il est représenté par l'un des composés de formule :

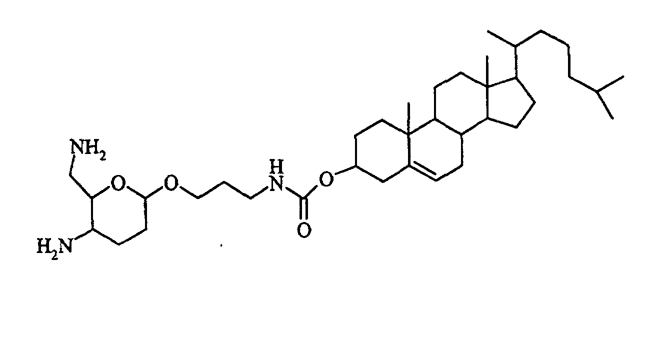

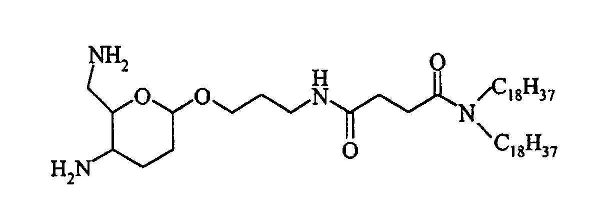

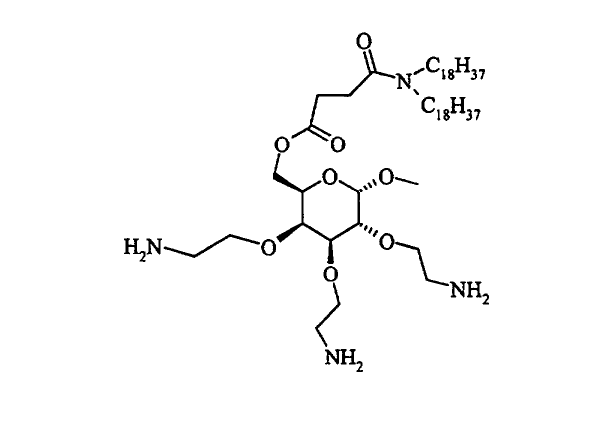

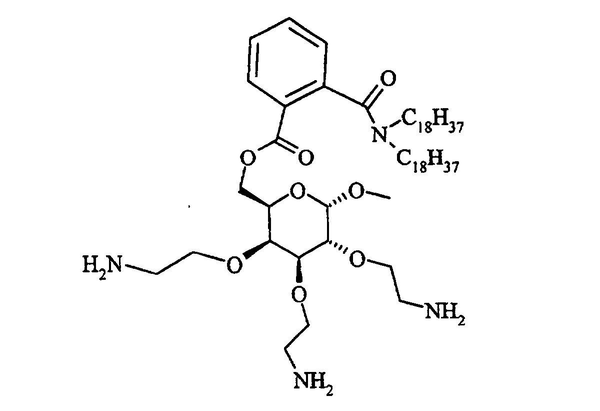

13. Procédé de préparation d'un agent transfectant selon les revendications 1 à 12 **caractérisé en ce que** l'on couple la région lipophile par l'intermédiaire d'un "spacer" à l'hétérocycle de formule générale telle que définie dans la revendication 1.

14. Composition **caractérisée en ce qu'**elle comprend un agent transfectant tel que défini dans les revendications 1 à 12 et au moins un acide nucléique.

15. Composition selon la revendication 21 **caractérisée en ce que** le rapport agent transfectant/acide nucléique est compris entre 0,1 et 50 nanomoles d'agent par µg d'ADN

16. Composition selon la revendication 14 **caractérisée en ce qu'**elle incorpore en outre un ou plusieurs adjuvants.

17. Composition selon la revendication 16 **caractérisée en ce que** le ou les adjuvants sont des lipides neutres.

18. Composition selon la revendication 16 **caractérisée en ce que** le ou les adjuvants sont sont des composés intervenant directement ou non au niveau de la condensation de l'acide nucléique.

19. Composition selon les revendications 14 à 18 **caractérisée en ce qu'**elle comprend un véhicule pharmaceutiquement acceptable pour une formulation injectable.

20. Composition selon les revendications 14 à 18 **caractérisée en ce qu'**elle comprend un véhicule pharmaceutiquement acceptable pour une application sur la peau et/ou les muqueuses.

21. Méthode de transfert d'acides nucléiques dans les cellules **caractérisée en ce qu'**elle comprend les étapes suivantes :
(1) la mise en contact de l'acide nucléique avec un agent de transfert tel que défini dans les revendications 1 à 19 et, le cas échéant, avec le ou les adjuvants, pour former un complexe nucléolipidique, et
(2) la mise en contact des cellules avec le complexe formé en (1).

22. Agent transfectant selon les revendications 1 à 13 pour son utilisation dans le transfert d'acides nucléiques dans les cellules.

23. Utilisation d'un agent transfectant tel que défini dans les revendications précédentes pour la fabrication d'un médicament contenant au moins un acide nucléique à transfecter.

## Patentansprüche

1. Transfektionsmittel, umfassend mindestens eine hydrophile kationische Region in Kopplung an eine lipophile Region, **dadurch gekennzeichnet, dass** die hydrophile kationische Region die allgemeine Formel 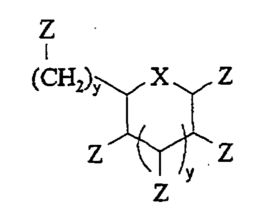 besitzt, worin
- y eine ganze Zahl von 0 oder 1 ist, wobei die verschiedenen y voneinander unabhängig sind,
- X ein Sauerstoff-, Stickstoff-, Schwefel- oder Selenatom bezeichnet,
- die Gruppierungen Z unabhängig voneinander
. ein Wasserstoffatom,
. eine OR-Gruppierung, worin R ein Wasserstoffatom, eine Methylgruppe oder eine Gruppierung (CH₂)ₙ-NR₁R₂ ist, - worin n eine ganze Zahl ausgewählt aus 1 bis 6 einschießlich ist und R₁ und R₂ unabhängig voneinander ein Wasserstoffatom oder eine Gruppierung (CH₂)q-NH₂ bezeichnen, wobei q von 1 bis 6 einschließlich variieren kann und die verschiedenen q voneinander unabhängig sind,
. eine Gruppierung (CH₂)m-NR₁R₂, worin m eine ganze Zahl ausgewählt aus 1 bis 6 einschießlich ist und R₁ und R₂ wie vorstehend definiert sind, oder
. eine Spacer-Gruppierung, die die Bindung der hydrophilen kationischen Region an die lipophile Region erlaubt,
bedeuten,
mit der Maßgabe, dass mindestens einer der Substituenten Z eine Spacer-Gruppierung ist und mindestens zwei der Substituenten Z eine Aminogruppe tragen.

2. Transfektionsmittel nach Anspruch 1, **dadurch gekennzeichnet, dass** in der allgemeinen Formel, die die hydrophilen kationischen Region definiert, n eine ganze Zahl ausgewählt aus 2,3 und 4 in der Gruppierung (CH₂)n-NR₁R₂ ist.

3. Transfektionsmittel nach Anspruch 1, **dadurch gekennzeichnet, dass** die allgemeine Formel, die die hydrophile kationische Region definiert, ein Glykosid der Form Pyranose oder Furanose ist.

4. Transfektionsmittel nach Anspruch 1, **dadurch gekennzeichnet, dass** in der allgemeinen Formel, die die hydrophile kationische Region definiert, y gleich 1 ist und X ein Sauerstoffatom ist.

5. Transfektionsmittel nach Anspruch 1, **dadurch gekennzeichnet, dass** die lipophile Region aus einer oder mehreren linearen oder verzweigten gesättigten oder nicht-gesättigten und gegebenenfalls halogenierten aliphatischen Kette(n) und/oder aus einem Steroidderivat besteht.

6. Transfektionsmittel nach Anspruch 5, **dadurch gekennzeichnet, dass** die aliphatischen Ketten 10 bis 22 Kohlenstoffatome und insbesondere 14, 16, 17, 18 und/oder 19 Kohlenstoffatome enthalten.

7. Transfektionsmittel nach den Ansprüchen 5 und 6, **dadurch gekennzeichnet, dass** die aliphatischen Ketten ausgewählt sind aus (CH₂)₁₃CH₃, (CH₂)₁₅CH₃, (CH₂)₁₆CH₃, (CH₂)₁₇CH₃ und (CH₂)₁₈CH₃.

8. Transfektionsmittel nach Anspruch 5, **dadurch gekennzeichnet, dass** das Steroidderivat ausgewählt ist aus Cholesterol, Cholestanol, 3α,5-Cyclo-5α-cholestan-6β-ol, Cholsäure, Cholesterylformiat, Cholestanylformiat, 3α,5-Cyclo-5α-cholestan-6β-ylformiat, Cholesterylamin, 6-(1,5-Dimethylhexyl)-3a,5a-dimethylhexadecahydrocyclopenta[a]-cyclopropa[2,3]cyclopenta[1,2-f]naphthalen-10-ylamin oder Cholestanylamin.

9. Transfektionsmittel nach Anspruch 1, **dadurch gekennzeichnet, dass** die Spacer-Gruppierung, die die Bindung der kationischen hydrophilen Region an die lipophile Region erlaubt, aus einer Säure- oder Amingruppe besteht, die hydrolysierbare Funktionen trägt.

10. Transfektionsmittel nach Anspruch 9, **dadurch gekennzeichnet, dass** die Spacer-Gruppierung aus einer aliphatischen oder aromatischen Kette besteht und eine oder mehrere Gruppierung(en) ausgewählt aus Amiden, Estern, Ethern, Carbamaten und aromatischen Ringen trägt.

11. Transfektionsmittel nach Anspruch 9, **dadurch gekennzeichnet, dass** die Spacer-Gruppierung ausgewählt ist aus -O-CO-(CH₂)ₓ-COOH, -O-(CH₂)ₓ-COOH, -O-CO-(CH₂)ₓ-NH₂, -O-(CH₂)ₓ-NH₂, -NH-(CH₂)ₓ-NH₂, wobei x eine ganze Zahl zwischen 1 und 6 einschließlich bezeichnet.

12. Transfektionsmittel nach Anspruch 1, **dadurch gekennzeichnet, dass** es durch eine der folgenden Formeln widergegeben wird:

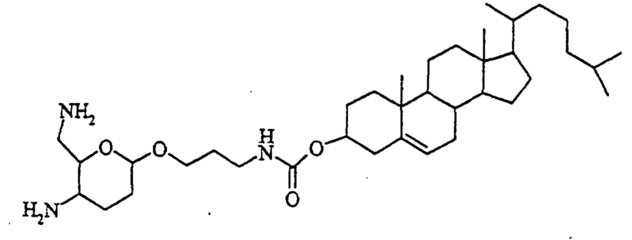

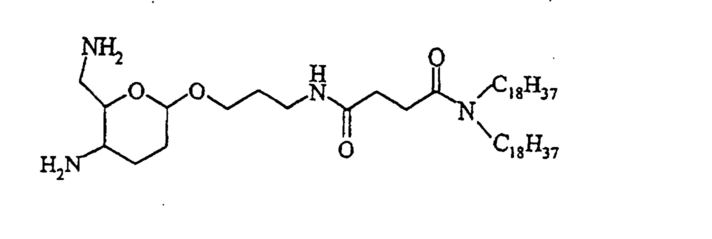

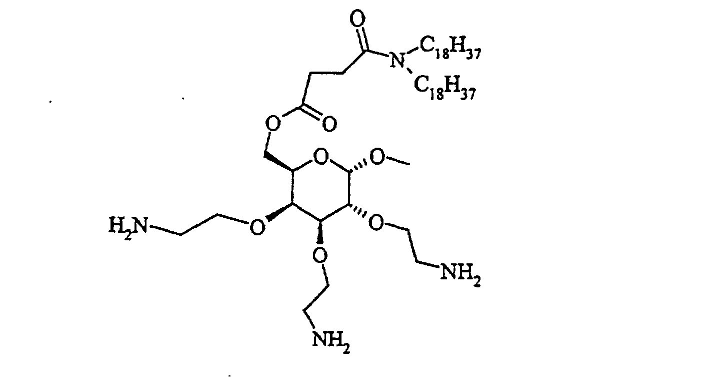

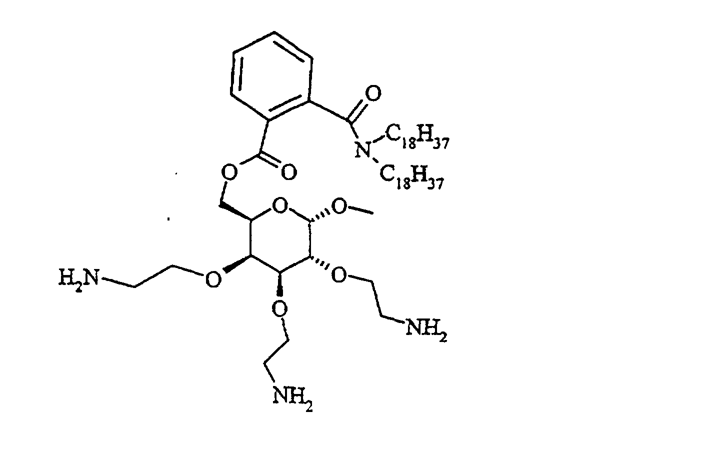

13. Verfahren zur Herstellung eines Transfektionsmittels nach einem der Ansprüche 1 bis 12, **dadurch gekennzeichnet, dass** man die lipophile Region über die Vermittlung eines Spacers an einen Heterocyclus der allgemeinen Formel wie in Anspruch 1 definiert koppelt.

14. Zusammensetzung **dadurch gekennzeichnet, dass** sie ein Transfektiönsmittel wie in den Ansprüchen 1 bis 12 definiert und mindestens eine Nucleinsäure umfasst.

15. Zusammensetzung nach Anspruch 21, **dadurch gekennzeichnet, dass** das Verhältnis Transfektionsmittel/ Nucleinsäure zwischen 0,1 und 50 nmol Transfektionsmittel pro µg DNA liegt.

16. Zusammensetzung nach Anspruch 14, **dadurch gekennzeichnet, dass** ein oder mehrere Anjuvans/Adjuvantien eingearbeitet ist/sind.

17. Zusammensetzung nach Anspruch 16, **dadurch gekennzeichnet, dass** das Adjuvans/die Adjuvantien neutrale Lipide sind.

18. Zusammensetzung nach Anspruch 16, **dadurch gekennzeichnet, dass** das Adjuvans/die Adjuvantien Verbindungen sind, die direkt oder indirekt auf der Ebene der Kondensation der Nukleinsäuren einwirken.

19. Zusammensetzung nach den Ansprüchen 14 bis 18 **dadurch gekennzeichnet, dass** sie einen pharmazeutisch verträglichen Träger für eine injizierbare Formulierung umfasst.

20. Zusammensetzung nach den Ansprüchen 14 bis 18 **dadurch gekennzeichnet, dass** sie einen pharmazeutisch verträglichen Träger für eine Anwendung auf der Haut und/oder den Schleimhäuten umfasst.

21. Verfahren zum Transfer von Nucleinsäuren in Zellen, **dadurch gekennzeichnet, dass** es die folgenden Stufen umfasst:
(1) In-Kontakt-Bringen der Nucleinsäure mit einem Transfermittel wie in den Ansprüchen 1 bis 19 definiert, und gegebenenfalls mit dem Adjuvans/den Adjuvantien zur Bildung eines Nucleolipidkomplexes, und
(2) In-Kontakt-Bringen der Zellen mit dem in (1) gebildeten Komplex.

22. Transfektionsmittel nach den Ansprüchen 1 bis 13 zur Verwendung zum Transfer von Nucleinsäuren in Zellen.

23. Verwendung eines Transfektionsmittels wie in einem der vorstehenden Ansprüche definiert zur Herstellung eines Medikaments, das mindestens eine zu transfizierende Nucleinsäure enthält.

## Claims

1. Transfecting agent comprising at least one cationic hydrophilic region coupled to a lipophilic region, **characterized in that** the said cationic hydrophilic region has the general formula: 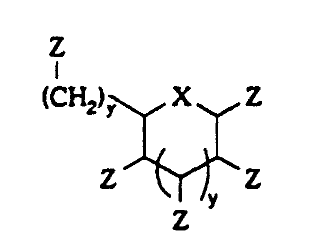 in which:
- y is an integer equal to 0 or 1, the different y's being independent from each other,
- X represents an oxygen, nitrogen, sulphur or selenium atom,
- the Z groups represent, independently of each other,
• a hydrogen atom,
• an OR group in which R represents a hydrogen atom, a methyl group or a group (CH2)ₙ-NR₁R₂ in which n is an integer chosen from 1 to 6 inclusive and R₁ and R₂ represent, independently of each other, a hydrogen atom or a group (CH₂)q-NH₂, it being possible for q to vary from 1 to 6 inclusive, the different q's being independent of each other,
• a group (CH₂)ₘ-NR₁R₂, in which m is an integer chosen from 0 to 6 inclusive and R₁ and R₂ are as defined above, or alternatively
• a "spacer" group allowing the binding of the cationic hydrophilic region to the lipophilic region,
it being understood that at least one of the Z substituents is a "spacer" group, and that at least two of the Z substituents carry an amino group.

2. Transfecting agent according to Claim 1, **characterized in that**, in the general formula defining the cationic hydrophilic region, n is an integer chosen from 2, 3 and 4 in the group (CH2)ₙ-NR₁R₂.

3. Transfecting agent according to Claim 1, **characterized in that** the general formula defining the cationic hydrophilic region is a glycoside of the pyranose or furanose form.

4. Transfecting agent according to Claim 1, **characterized in that**, in the general formula defining the cationic hydrophilic region, y is equal to 1 and X is an oxygen atom.

5. Transfecting agent according to Claim 1, **characterized in that** the said lipophilic region consists of one or more optionally halogenated, saturated or unsaturated, linear or branched aliphatic chains and/or a steroid derivative.

6. Transfecting agent according to Claim 5, **characterized in that** the said aliphatic chains contain 10 to 22 carbon atoms, and in particular 14, 16, 17, 18 and/or 19 carbon atoms.

7. Transfecting agent according to Claims 5 and 6, **characterized in that** the said aliphatic chains are chosen from (CH₂)₁₃CH₃, (CH₂)₁₅CH₃, (CH₂)₁₆CH₃, (CH₂)₁₇CH₃ and (CH₂)₁₈CH₃.

8. Transfecting agent according to Claim 5, **characterized in that** the steroid derivative is chosen from cholesterol, cholestanol, 3-α-5-cylco-5-α-cholestan-6-β-ol, cholic acid, cholesteryl formate, cholestanyl formate, 3α-5-cyclo-5α-cholestan-6β-yl formate, cholesterylamine, 6-(1,5-dimethylhexyl)-3a,5a-dimethylhexadecahydrocyclopenta[a]cyclopropa[2,3]cyclopenta[1,2-f]naphthalen-10-ylamine or cholestanylamine.

9. Transfecting agent according to Claim 1, **characterized in that** the "spacer" group allowing the linkage of the cationic hydrophilic region to the lipophilic region consists of an acid or amino group comprising hydrolysable functional groups.

10. Transfecting agent according to Claim 9, **characterized in that** the "spacer" group consists of an aliphatic or aromatic chain and comprises one or more groups chosen from amides, esters, ethers, carbamates and aromatic rings.

11. Transfecting agent according to Claim 9, **characterized in that** the "spacer" is chosen from -O-CO-(CH₂)ₓ-COOH, -O-(CH₂)ₓ-COOH, -O-CO-(CH₂)ₓ-NH₂, -O-(CH₂)ₓ-NH₂, -NH- (CH₂)ₓ-NR₂, with x representing an integer between 1 and 6 inclusive.

12. Transfecting agent according to Claim 1, **characterized in that** it is represented by one of the compounds of formula:

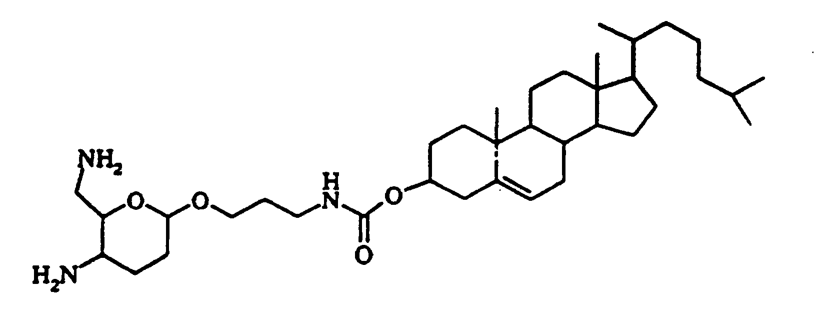

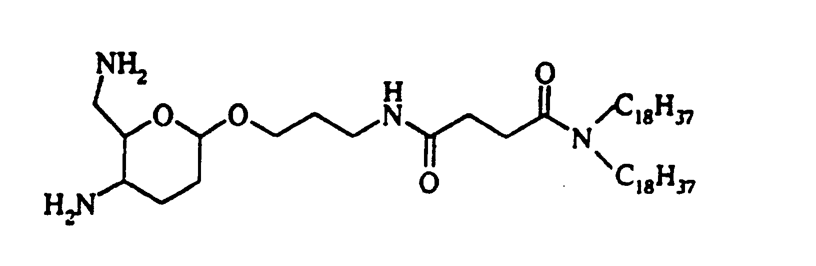

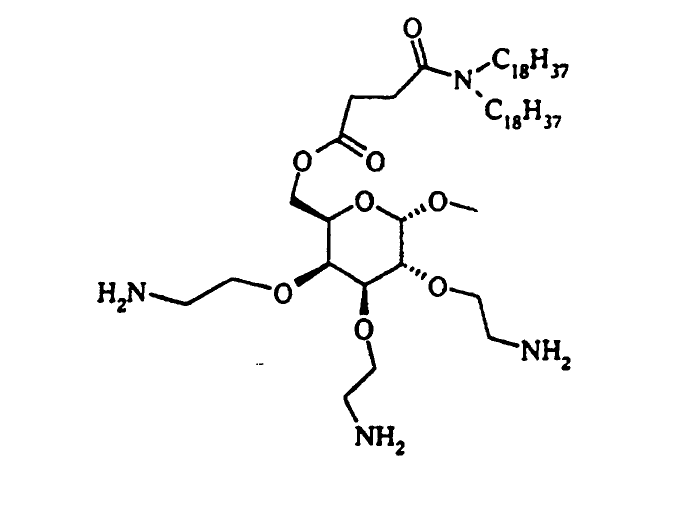

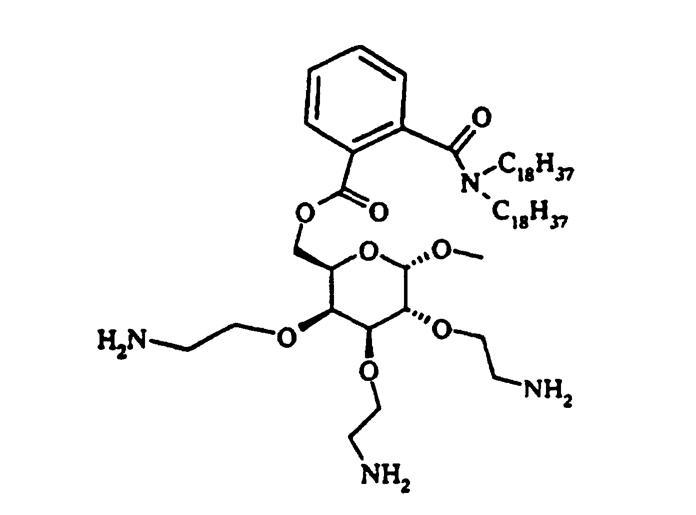

13. Method of preparing a transfecting agent according to Claims 1 to 12, **characterized in that** the lipophilic region is coupled by means of a "spacer" to the heterocycle of general formula as defined in Claim 1.

14. Composition **characterized in that** it comprises a transfecting agent as defined in Claims 1 to 12 and at least one nucleic acid.

15. Composition according to Claim 14, **characterized in that** the transfecting agent/nucleic acid ratio is between 0.1 and 50 nanomoles of agent per µg of DNA.

16. Composition according to Claim 14, **characterized in that** it incorporates, in addition, one or more adjuvants.

17. Composition according to Claim 16, **characterized in that** the adjuvant(s) is(are) a neutral lipid(s).

18. Composition according to Claim 16, **characterized in that** the adjuvant(s) is(are) a compound(s) which is(are) involved directly or indirectly in the condensation of the nucleic acid.

19. Composition according to Claims 14 to 18, **characterized in that** it comprises a pharmaceutically acceptable vehicle for an injectable formulation.

20. Composition according to Claims 14 to 18, **characterized in that** it comprises a pharmaceutically acceptable vehicle for application to the skin and/or the mucous membranes.

21. Method of transferring nucleic acids into cells, **characterized in that** it comprises the following steps:
(1) bringing the nucleic acid into contact with a transfer agent as defined in Claims 1 to 19 and, where appropriate, with the adjuvant(s), to form a nucleolipid complex, and
(2) bringing the cells into contact with the complex formed in (1).

22. Transfecting agent according to Claims 1 to 13 for its use in transferring nucleic acids into cells.

23. Use of a transfecting agent as defined in the preceding claims for manufacturing a medicament containing at least one nucleic acid to be transfected.
